(19) Europäisches Patentamt
European Patent Office
Office européen des brevets

(11) EP 1 838 309 B1

(12) # EUROPEAN PATENT SPECIFICATION

(45) Date of publication and mention of the grant of the patent:
**20.08.2008 Bulletin 2008/34**

(21) Application number: **05818222.1**

(22) Date of filing: **07.12.2005**

(51) Int Cl.:
***A61K 31/437*** *(2006.01)* ***C07D 471/04*** *(2006.01)*
***A61P 1/04*** *(2006.01)*

(86) International application number:
**PCT/IB2005/003758**

(87) International publication number:
**WO 2006/064339 (22.06.2006 Gazette 2006/25)**

(54) **CHROMANE DERIVATIVES USEFUL AS ACID PUMP ANTAGONISTS**

CHROMAN-DERIVATE ALS SÄUREPUMPENANTAGONISTEN

DERIVES DE CHROMANE UTILISES COMME ANTAGONISTES DE LA POMPE A ACIDE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **17.12.2004 US 636963 P**
**29.06.2005 US 695772 P**

(43) Date of publication of application:
**03.10.2007 Bulletin 2007/40**

(73) Proprietor: **Pfizer, Inc.**
**New York, NY 10017 (US)**

(72) Inventors:
- **JINNO, Madoka,**
  **Pfizer Japan Inc.**
  **Shibuya-ku,**
  **Tokyo 151-8589 (JP)**
- **SHIMOKAWA, Hirohisa,**
  **Pfizer Japan Inc.**
  **Shibuya-ku,**
  **Tokyo 151-8589 (JP)**
- **YAMAGISHI, Tatsuya,**
  **Pfizer Japan Inc.**
  **Shibuya-ku,**
  **Tokyo 151-8589 (JP)**

(74) Representative: **Rudge, Andrew John**
**Pfizer Limited**
**European Pharma Patent Department**
**Ramsgate Road**
**Sandwich, Kent CT13 9NJ (GB)**

(56) References cited:
**WO-A-99/55706** **WO-A-03/018582**
**WO-A-20/04046144**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Background of the Invention

This invention relates to chromane derivatives. These compounds have selective acid pump inhibitory activity. The present invention also relates to a pharmaceutical composition, and use, comprising the above derivatives for the treatment of disease conditions mediated by acid pump modulating activity; in particular acid pump inhibitory activity.

It has been well established that proton pump inhibitors (PPIs) are prodrugs that undergo an acid-catalyzed chemical rearrangement that permits them to inhibit $H^+/K^+$-ATPase by covalently biding to its Cystein residues (Sachs, G. et. al., Digestive Diseases and Sciences, 1995, 40, 3S-23S; Sachs et. al., Annu Rev Pharmacol Toxicol, 1995, 35, 277-305.). However, unlike PPIs, acid pump antagonists inhibit acid secretion via reversible potassium-competitive inhibition of $H^+/K^+$-ATPase. SCH28080 is one of such reversible inhibitors and has been studied extensively. Other newer agents (revaprazan, soraprazan, AZD-0865 and CS-526) have entered in clinical trials confirming their efficacy in human (Pope, A.; Parsons, M., Trends in Pharmacological Sciences, 1993,14, 323-5; Vakil, N., Alimentary Pharmacology and Therapeutics, 2004, 19, 1041-1049). In general, acid pump antagonists are found to be useful for the treatment of a variety of diseases, including gastrointestinal disease, gastroesophageal disease, gastroesophageal reflux disease (GERD), peptic ulcer, gastric ulcer, duodenal ulcer, non-steroidal anti-inflammatory drug (NSAID)-induced ulcers, gastritis, infection of Helicobacter pylori, dyspepsia, functional dyspepsia, Zollinger-Ellison syndrome, non-erosive reflux disease (NERD), visceral pain, heartburn, nausea, esophagitis, dysphagia, hypersalivation, airway disorders or asthma (hereinafter, referred as "APA Diseases", Kiljander, Toni O, American Journal of Medicine, 2003, 115 (Suppl. 3A), 65S-71S.).

WO99/55706 and WO04/046144 disclose compounds reported to be acid pump antagonists. They refer to certain compounds having imidazo[1,2-a]pyridine structure.

There is a need to provide new acid pump antagonists that are good drug candidates and address unmet needs by PPIs for treating diseases. In particular, preferred compounds should bind potently to the acid pump whilst showing little affinity for other receptors and show functional activity as inhibitors of acid-secretion in stomach. They should be well absorbed from the gastrointestinal tract, be metabolically stable and possess favorable pharmacokinetic properties. They should be non-toxic. Furthermore, the ideal drug candidate will exist in a physical form that is stable, non-hygroscopic and easily formulated.

### Summary of the Invention

In this invention, it has now been found out that the new class of compounds having a chromane moiety show acid pump inhibitory activity and favorable properties as drug candidates, and thus are useful for the treatment of disease conditions mediated by acid pump inhibitory activity such as APA Diseases.

The present invention provides a compound of the following formula (I):

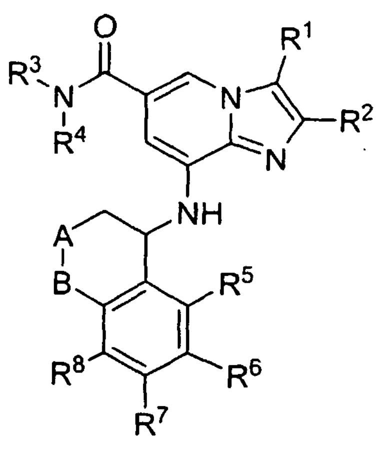

(I)

or a pharmaceutically acceptable salt thereof, wherein:

-A-B- represents $-O-CH_2-$, $-S-CH_2-$, $-CH_2-O-$ or $-CH_2-S-$;
$R^1$ represents a $C_1$-$C_6$ alkyl group being unsubstituted or substituted with 1 to 2 substituents independently selected from the group consisting of a hydroxy group, a moiety convertible in vivo into a hydroxy group and a $C_1$-$C_6$ alkoxy

group;
$R^2$ represents a $C_1$-$C_6$ alkyl group;
$R^3$ represents a $C_1$-$C_6$ alkyl group, $C_3$-$C_7$cycloalkyl group or $C_3$-$C_7$ cycloalkyl $C_1$-$C_6$ alkyl group;
$R^4$ represents a $C_1$-$C_6$ alkyl group being unsubstituted or substituted with 1 to 3 substituents independently selected from the group consisting of a halogen atom, a hydroxy group, a moiety convertible in vivo into a hydroxy group and a $C_1$-$C_6$ alkoxy group; and
$R^5$, $R^6$, $R^7$ and $R^8$ independently represent a hydrogen atom, a halogen atom or a $C_1$-$C_6$ alkyl group where in the moiety convertible in vivo into a hydroxy group on a $C_1$-$C_6$ alkyl Carbonyl oxy group on a $C_1$-$C_6$ alkyl carbonyl oxy methyl oxy group.

Also, the present invention provides a pharmaceutical composition comprising a compound of formula (I) or a pharmaceutically acceptable salt thereof, each as described herein, together with a pharmaceutically acceptable carrier for said compound.

Also, the present invention provides a pharmaceutical composition comprising a compound of formula (I) or a pharmaceutically acceptable salt thereof, each as described herein, further comprising other pharmacologically active agent(s).

A method of treatment of a condition mediated by acid pump modulating activity, in a mammalian subject comprises administering to a mammal in need of such treatment a therapeutically effective amount of a compound of formula (I) or a pharmaceutically acceptable salt thereof, each as described herein.

Examples of conditions mediated by acid pump modulating activity include, but are not limited to, APA Diseases.

Further, the present invention provides the use of a compound of formula (I) or a pharmaceutically acceptable salt thereof, each as described herein, for the manufacture of a medicament for the treatment of a condition mediated by acid pump inhibitory activity.

Preferably, the present invention also provides the use of a compound of formula (I) or a pharmaceutically acceptable salt thereof, each as described herein, for the manufacture of a medicament for the treatment of diseases selected from APA Diseases.

The compounds of the present invention may show good acid pump inhibitory activity, less toxicity, good absorption, good distribution, good solubility, less protein binding affinity other than acid pump, less drug-drug interaction, and good metabolic stability.

Some stereoisomers of the present invention may show a better property of phototoxicity.

## Detailed Description of the Invention

In the compounds of the present invention:

Where $R^1$, $R^2$, $R^3$, $R^4$, $R^5$, $R^6$, $R^7$ and $R^8$ are the $C_1$-$C_6$ alkyl group, this $C_1$-$C_6$ alkyl group may be a straight or branched chain group having one to six carbon atoms, and examples include, but are not limited to, a methyl, ethyl, propyl, isopropyl, butyl, isobutyl, sec-butyl and *tert*-butyl, pentyl, 1-ethylpropyl and hexyl. Of these, $C_1$-$C_4$ alkyl is preferred; $C_1$-$C_2$ alkyl is more preferred; methyl is preferred for $R^1$, $R^2$, $R^3$, $R^5$, $R^6$, $R^7$ and $R^8$; methyl and ethyl are preferred for $R^4$.
Where $R^3$ is the $C_3$-$C_7$ cycloalkyl group, this represents cycloalkyl group having three to seven carbon atoms, and examples include cyclopropyl, cyclobutyl, cyclopentyl, cyclohexyl and cycloheptyl. Of these, $C_3$-$C_5$ cycloalkyl group is preferred; cyclopropyl is more preferred.
Where $R^3$ is the $C_3$-$C_7$ cycloalkyl $C_1$-$C_6$ alkyl group, this represents the said $C_1$-$C_6$ alkyl group substituted with the said $C_3$-$C_7$ cycloalkyl group, and examples include, but are not limited to, cyclopropylmethyl, cyclopropylethyl, cyclopropylpropyl, cyclopropylbutyl, cyclopropylpentyl, cyclopropylhexyl, cyclobutylmethyl, cyclobutylethyl, cyclopentylmethyl, cyclohexylmethyl and cycloheptylmethyl. Of these, $C_3$-$C_5$ cycloalkyl $C_1$-$C_4$ alkyl group is preferred; $C_3$-$C_5$ cycloalkyl $C_1$-$C_2$ alkyl group is preferred; cyclopropylmethyl is more preferred.
Where the substituents of $R^1$ and $R^4$ are the $C_1$-$C_6$ alkoxy group, this represents the oxygen atom substituted with the said $C_1$-$C_6$ alkyl group, and examples include, but are not limited to, methoxy, ethoxy, propoxy, isopropoxy, butoxy, isobutoxy, sec-butoxy and *tert*-butoxy, pentyloxy and hexyloxy. Of these, a $C_1$-$C_4$ alkyloxy is preferred; a $C_1$-$C_2$ alkyloxy is preferred; methoxy is more preferred.
Where $R^5$, $R^6$, $R^7$ and $R^8$ are the halogen atom, they may be a fluorine, chlorine, bromine or iodine atom. Of these, a fluorine atom and a chlorine atom are preferred.
Where -A-B- is -O-$CH_2$- or -S-$CH_2$-, -A- corresponds -O or -S- and -B- corresponds -$CH_2$-.
Where -A-B- is -$CH_2$-O- or -$CH_2$-S -, -A- corresponds -$CH_2$- and -B- corresponds -O- or -S-.

The term **"treating"** and **"treatment",** as used herein, refers to curative, palliative and prophylactic treatment,

including reversing, alleviating, inhibiting the progress of, or preventing the disorder or condition to which such term applies, or one or more symptoms of such disorder or condition.

Preferred class of compounds of the present invention are those compounds of formula (I) or a pharmaceutically acceptable salt thereof, each as described herein, in which:

(a) -A-B- is $-O-CH_2-$ or $-CH_2-O-$;
(b) $R^1$ is a $C_1$-$C_4$ alkyl group;
(c) $R^1$ is a $C_1$-$C_2$ alkyl group;
(d) $R^1$ is a methyl group;
(e) $R^2$ is a $C_1$-$C_2$ alkyl group;
(f) $R^2$ is a methyl group;
(g) $R^3$ is a $C_1$-$C_4$ alkyl group;
(h) $R^3$ is a $C_1$-$C_2$ alkyl group;
(i) $R^3$ is a methyl group;
(j) $R^4$ is a $C_1$-$C_4$ alkyl group being unsubstituted or substituted with 1 substituent selected from the group consisting of a hydroxy group and a $C_1$-$C_4$ alkoxy group;
(k) $R^4$ is a $C_1$-$C_2$ alkyl group being unsubstituted or substituted with 1 substituent selected from the group consisting of a hydroxy group and a $C_1$-$C_4$ alkoxy group;
(l) $R^4$ is a $C_1$-$C_2$ alkyl group being unsubstituted or substituted with a hydroxy group;
(m) $R^4$ is a methyl group, an ethyl group or a 2-hydroxyethyl group;
(n) $R^5$, $R^6$, $R^7$ and $R^8$ are independently a hydrogen atom, a halogen atom or a $C_1$-$C_2$ alkyl group;
(o) $R^5$, $R^6$ $R^7$ and $R^8$ are independently a hydrogen atom, a fluorine atom, a chlorine atom, or a methyl group;
(p) $R^5$, $R^6$, $R^7$ and $R^8$ are independently a hydrogen atom or a methyl group
(q) $R^5$ is a hydrogen atom, a fluorine atom or a methyl group;
(r) $R^6$ is a hydrogen atom;
(s) $R^7$ is a hydrogen atom or a fluorine atom; and
(t) $R^8$ is a hydrogen atom;

Of these classes of compounds, any combination among (a) to (t) is also preferred.

Preferred compounds of the present invention are those compounds of formula (I) or a pharmaceutically acceptable salt thereof, each as described herein, in which:

(A) -A-B- is $-O-CH_2-$, $-S-CH_2-$, $-CH_2-O-$ or $-CH_2-S-$; $R^1$ is a $C_1$-$C_4$ alkyl group being unsubstituted or substituted with 1 to 2 substituents independently selected from the group consisting of a hydroxy group and a $C_1$-$C_4$ alkoxy group; $R^2$ is a $C_1$-$C_4$ alkyl group; $R^3$ is a $C_1$-$C_4$ alkyl group, $C_3$-$C_7$ cycloalkyl group or $C_3$-$C_7$ cycloalkyl $C_1$-$C_4$ alkyl group; $R^4$ is a $C_1$-$C_4$ alkyl group being unsubstituted or substituted with 1 to 3 substituents independently selected from the group consisting of a halogen atom, a hydroxy group and a $C_1$-$C_4$ alkoxy group; and $R^5$, $R^6$, $R^7$ and $R^8$ are independently a hydrogen atom, a halogen atom or a $C_1$-$C_6$ alkyl group;
(B) -A-B- is $-O-CH_2-$, or $-CH_2-O-$; $R^1$, $R^2$ and $R^3$ are independently a $C_1$-$C_4$ alkyl group; $R^4$ is a $C_1$-$C_4$ alkyl group being unsubstituted or substituted with 1 substituent selected from the group consisting of a hydroxy group and a $C_1$-$C_4$ alkoxy group; $R^5$ and $R^7$ are independently a hydrogen atom, a halogen atom or a $C_1$-$C_4$ alkyl group; and $R^6$ and $R^8$ are each hydrogen atom;
(C) -A-B- is $-O-CH_2-$, or $-CH_2-O-$; $R^1$, $R^2$ and $R^3$ are independently a $C_1$-$C_2$ alkyl group; $R^4$ is a $C_1$-$C_2$ alkyl group being unsubstituted or substituted with a hydroxy group; $R^5$ and $R^7$ are independently a hydrogen atom or a $C_1$-$C_2$ alkyl group; and $R^6$ and $R^8$ are each hydrogen atom;
(D) -A-B- is $-O-CH_2-$, or $-CH_2-O-$; $R^1$ is a $C_1$-$C_4$ alkyl group being unsubstituted or substituted with 1 to 2 substituents independently selected from the group consisting of a hydroxy group and a $C_1$-$C_4$ alkoxy group; $R^2$ is a $C_1$-$C_4$ alkyl group; $R^3$ is a $C_1$-$C_4$ alkyl group, $C_3$-$C_7$ cycloalkyl group or $C_3$-$C_7$ cycloalkyl $C_1$-$C_4$ alkyl group; $R^4$ is a $C_1$-$C_4$ alkyl group being unsubstituted or substituted with 1 to 3 substituents independently selected from the group consisting of a halogen atom, a hydroxy group and a $C_1$-$C_4$ alkoxy group; $R^5$ and $R^7$ are independently a hydrogen atom, a halogen atom or a $C_1$-$C_4$ alkyl group; and $R^6$ and $R^8$ are each hydrogen atom;
(E) -A-B- is $-O-CH_2-$, $-S-CH_2-$, $-CH_2-O-$ or $-CH_2-S-$; $R^1$ and $R^2$ are independently a $C_1$-$C_4$ alkyl group; $R^3$ is a $C_1$-$C_4$ alkyl group, $C_3$-$C_7$ cycloalkyl group or $C_3$-$C_7$ cycloalkyl $C_1$-$C_4$ alkyl group; $R^4$ is a $C_1$-$C_4$ alkyl group being unsubstituted or substituted with 1 to 3 substituents independently selected from the group consisting of a halogen atom, a hydroxy group and a $C_1$-$C_4$ alkoxy group; $R^5$ and $R^7$ are independently a hydrogen atom, a halogen atom or a $C_1$-$C_4$ alkyl group; and $R^6$ and $R^8$ are each hydrogen atom;
(F) -A-B- is $-O-CH_2-$, $-S-CH_2-$, $-CH_2-O-$ or $-CH_2-S-$; $R^1$ and $R^2$ are each methyl group; $R^3$ is a $C_1$-$C_4$ alkyl group, $C_3$-$C_7$ cycloalkyl group or $C_3$-$C_7$ cycloalkyl $C_1$-$C_4$ alkyl group; $R^4$ is a $C_1$-$C_4$ alkyl group being unsubstituted or

substituted with 1 to 3 substituents independently selected from the group consisting of a halogen atom, a hydroxy group and a $C_1$-$C_4$ alkoxy group; $R^5$ and $R^7$ are independently a hydrogen atom, a halogen atom or a $C_1$-$C_4$ alkyl group; and $R^6$ and $R^8$ are each hydrogen atom;

(G) -A-B- is -O-$CH_2$-, -S-$CH_2$-, -$CH_2$-O- or -$CH_2$-S-; $R^1$ and $R^2$ are each methyl group; $R^3$ is a $C_1$-$C_4$ alkyl group, $C_3$-$C_7$ cycloalkyl group or $C_3$-$C_7$ cycloalkyl $C_1$-$C_4$ alkyl group; $R^4$ is a $C_1$-$C_4$ alkyl group being unsubstituted or substituted with 1 to 3 substituents independently selected from the group consisting of a halogen atom, a hydroxy group and a $C_1$-$C_4$ alkoxy group; $R^5$ and $R^7$ are independently a hydrogen atom, a halogen atom a or a $C_1$-$C_2$ alkyl group; and $R^6$ and $R^8$ are each hydrogen atom;

(H) -A-B- is -O-$CH_2$-, -S-$CH_2$-, -$CH_2$-O- or -$CH_2$-S-; $R^1$ and $R^2$ are each methyl group; $R^3$ is a $C_1$-$C_4$ alkyl group; $R^4$ is a $C_1$-$C_4$ alkyl group being unsubstituted or substituted 1 substituent selected from the group consisting of a hydroxy group and a $C_1$-$C_4$ alkoxy group; $R^5$ and $R^7$ are independently a hydrogen atom, a halogen atom or a methyl group; and $R^6$ and $R^8$ are each hydrogen atom;

(I) -A-B- is -O-$CH_2$-, -S-$CH_2$-, -$CH_2$-O- or -$CH_2$-S-; $R^1$ and $R^2$ are each methyl group; $R^3$ is a $C_1$-$C_2$ alkyl group; $R^4$ is a $C_1$-$C_2$ alkyl group being unsubstituted or substituted with a hydroxy group; $R^5$ and $R^7$ are independently a hydrogen atom or a methyl group; and $R^6$ and $R^8$ are each hydrogen atom;

(J) -A-B- is -O-$CH_2$-, or -$CH_2$-O-; $R^1$, $R^2$ and $R^3$ are independently a $C_1$-$C_4$ alkyl group; $R^4$ is a $C_1$-$C_4$ alkyl group being unsubstituted or substituted with 1 substituent selected from the group consisting of a hydroxy group and a $C_1$-$C_4$ alkoxy group; and $R^5$, $R^6$, $R^7$ and $R^8$ are independently a hydrogen atom, a halogen atom or a $C_1$-$C_4$ alkyl group;

(K) -A-B- is -$CH_2$-O-; $R^1$, $R^2$ and $R^3$ are independently a methyl group; $R^4$ is a $C_1$-$C_2$ alkyl group being unsubstituted or substituted with a hydroxy group; $R^5$ and $R^7$ are independently a hydrogen atom or methyl group; and $R^6$ and $R^8$ are each hydrogen atom;

(L) -A-B- is -O-$CH_2$-, or -$CH_2$-O-; $R^1$, $R^2$ and $R^3$ are independently a $C_1$-$C_6$ alkyl group; $R^4$ is a $C_1$-$C_6$ alkyl group being unsubstituted or substituted with 1 substituent selected from the group consisting of a hydroxy group and a $C_1$-$C_6$ alkoxy group; $R^5$ is a hydrogen atom, a fluorine atom or a $C_1$-$C_6$ alkyl group; $R^7$ is a hydrogen atom, a halogen atom or a $C_1$-$C_6$ alkyl group; $R^6$ and $R^8$ are independently a hydrogen atom, a halogen atom or a $C_1$-$C_6$ alkyl group;

(M) A-B- is -O-$CH_2$-, or -$CH_2$-O-; $R^1$, $R^2$ and $R^3$ are independently a $C_1$-$C_6$ alkyl group; $R^4$ is a $C_1$-$C_6$ alkyl group being unsubstituted or substituted with 1 substituent selected from the group consisting of a hydroxy group and a $C_1$-$C_6$ alkoxy group; $R^5$ is a hydrogen atom, a fluorine atom or a $C_1$-$C_6$ alkyl group; $R^7$ is a hydrogen atom or a halogen atom; and $R^6$ and $R^8$ are independently a hydrogen atom, a halogen atom or a $C_1$-$C_6$ alkyl group.

The compounds of formula (I) containing one or more asymmetric carbon atoms can exist as two or more stereoisomers.

Included within the scope of the present invention are all stereoisomers and geometric isomers of the compounds of formula (I), including compounds exhibiting more than one type of isomerism, and mixtures of one or more thereof. Also included are acid addition salts wherein the counterion is optically active, for example, D-lactate or L-lysine, or racemate, DL-tartrate or DL-arginine.

One embodiment of the invention provides a compound selected from the group consisting of:

(-)-8-(3,4-Dihydro-2*H*-chromen-4-ylamino)-*N*,*N*,2,3-tetramethylimidazo[1,2-*a*]pyridine-6-carboxamide;

(+)-8-(3,4-Dihydro-2*H*-chromen-4-ylamino)-*N*,*N*,2,3-tetramethylimidazo[1,2-*a*]pyridine-6-carboxamide;

(+)-8-(3,4-Dihydro-2*H*-chromen-4-ylamino)-*N*-(2-hydroxyethyl)-*N*,2,3-trimethylimidazo[1,2-*a*]pyridine-6-car boxamide;

(+)-*N*-(2-Hydroxyethyl)-*N*,2,3-trimethyl-8-[(5-methyl-3,4-dihydro-2*H*-chromen-4-yl)amino]imidazo[1,2-*a*]pyr idine-6-carboxamide;

(-)-8-[(7-Fluoro-3,4-dihydro-2*H*-chromen-4-yl)amino]-*N*,*N*,2,3-tetramethylimidazo[1,2-*a*]pyridine-6-carboxa mide;

(-)-8-[(7-Fluoro-3,4-dihydro-2*H*-chromen-4-yl)amino]-*N*-(2-hydroxyethyl)-*N*,2,3-trimethylimidazo[1,2-*a*]pyri dine-6-carboxamide;

(+)-8-[(7-Fluoro-3,4-dihydro-2*H*-chromen-4-yl)amino]-*N*-(2-hydroxyethyl)-*N*,2,3-trimethylimidazo[1,2-*a*]pyri dine-6-carboxamide;

(-)-8-[(5,7-Difluoro-3,4-dihydro-2*H*-chromen-4-yl)amino]-*N*,*N*,2,3-tetramethylimidazo[1,2-*a*]pyridine-6-carb oxamide;

(+)-8-[(5,7-Difluoro-3,4-dihydro-2*H*-chromen-4-yl)amino]-*N*,*N*,2,3-tetramethylimidazo[1,2-*a*]pyridine-6-carb oxamide;

(+)-8-[(5,7-Difluoro-3,4-dihydro-2*H*-chromen-4-yl)amino]-*N*-(2-hydroxyethyl)-*N*,2,3-trimethylimidazo[1,2-*a*] pyridine-6-carboxamide;

(-)-8-[(5-Fluoro-3,4-dihydro-2*H*-chromen-4-yl)amino]-*N*,*N*,2,3-tetramethylimidazo[1,2-*a*]pyridine-6-carboxa mide;

(+)-8-[(5-Fluoro-3,4-dihydro-2*H*-chromen-4-yl)amino]-*N*,*N*,2,3-tetramethylimidazo[1,2-*a*]pyridine-6-carbox amide;

(-)-8-[(5-Fluoro-3,4-dihydro-2*H*-chromen-4-yl)amino]-*N*-(2-hydroxyethyl)-*N*,2,3-trimethylimidazo[1,2-*a*]pyri dine-6-carboxamide;
(+)-8-[(5-Fluoro-3,4-dihydro-2*H*-chromen-4-yl)amino]-*N*-(2-hydroxyethyl)-*N*,2,3-trimethylimidazo[1,2-*a*]pyridine-6-carboxamide;
(-)-8-[(6-Fluoro-3,4-dihydro-2*H*-chromen-4-yl)amino]-*N,N*,2,3-tetramethylimidazo[1,2-*a*]pyridine-6-carboxamide;
(+)-8-[(6-Fluoro-3,4-dihydro-2*H*-chromen-4-yl)amino]-*N*-(2-hydroxyethyl)-*N*,2,3-trimethylimidazo[1,2-*a*]pyridine-6-carboxamide;
(-)-8-[(8-Fluoro-3,4-dihydro-2*H*-chromen-4-yl)amino]-*N,N*,2,3-tetramethylimidazo[1,2-*a*]pyridine-6-carboxamide;
(+)-8-[(8-Fluoro-3,4-dihydro-2*H*-chromen-4-yl)amino]-*N,N*,2,3-tetramethylimidazo[1,2-*a*]pyridine-6-carboxamide;
(-)-*N*-(2-Hydroxyethyl)-*N*,2,3-trimethyl-8-[(7-methyl-3,4-dihydro-2*H*-chromen-4-yl)amino]imidazo[1,2-*a*]pyridine-6-carboxamide;
(+)-*N*-(2-Hydroxyethyl)-*N*,2,3-trimethyl-8-[(7-methyl-3,4-dihydro-2*H*-chromen-4-yl)amino]imidazo[1,2-*a*]pyridine-6-carboxamide;
(-)-*N,N*,2,3-Tetramethyl-8-[(5-methyl-3,4-dihydro-2*H*-chromen-4-yl)amino]imidazo[1,2-*a*]pyridine-6-carboxamide; and
(+)-*N,N*,2,3-Tetramethyl-8-[(5-methyl-3,4-dihydro-2*H*-chromen-4-yl)amino]imidazo[1,2-*a*]pyridine-6-carboxamide;

or a pharmaceutically acceptable salt thereof.

Preferred stereoisomers of the compounds of formula (I) are R form with respect to a chiral center where the carbon atom on the chromane ring binds to the nitrogen atom.

Pharmaceutically acceptable salts of a compound of formula (I) include the acid addition salts (including disalts) thereof.

Suitable acid addition salts are formed from acids which form non-toxic salts. Examples include the acetate, adipate, aspartate, benzoate, besylate, bicarbonate/carbonate, bisulphate/sulphate, borate, camsylate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

For a review on suitable salts, see "Handbook of Pharmaceutical Salts: Properties, Selection, and Use" by Stahl and Wermuth (Wiley-VCH, Weinheim, Germany, 2002). A pharmaceutically acceptable salt of a compound of formula (I) may be readily prepared by mixing together solutions of the compound of formula (I) and the desired acid or base, as appropriate. The salt may precipitate from solution and be collected by filtration or may be recovered by evaporation of the solvent. The degree of ionization in the salt may vary from completely ionized to almost non-ionized.

Pharmaceutically acceptable salts of the compounds of the invention include both unsolvated and solvated forms. The term "solvate" is used herein to describe a molecular complex comprising a compound of the invention and one or more pharmaceutically acceptable solvent molecules, for example, ethanol. The term 'hydrate' is employed when said solvent is water.

Pharmaceutically acceptable solvates in accordance with the invention include hydrates and solvates wherein the solvent of crystallization may be isotopically substituted, e.g. $D_2O$, $d_6$-acetone, $d_6$-DMSO.

Included within the scope of the invention are complexes such as clathrates, drug-host inclusion complexes wherein, in contrast to the aforementioned solvates, the drug and host are present in stoichiometric or non-stoichiometric amounts. Also included are complexes of the drug containing two or more organic and/or inorganic components which may be in stoichiometric or non-stoichiometric amounts. The resulting complexes may be ionized, partially ionized, or non-ionized. For a review of such complexes, see J Pharm Sci, 64 (8), 1269-1288 by Haleblian (August 1975).

The compounds of formula (I) may exist in one or more crystalline forms. These polymorphs, including mixtures thereof are also included within the scope of the present invention.

The present invention includes all pharmaceutically acceptable isotopically-labeled compounds of formula (I) wherein one or more atoms are replaced by atoms having the same atomic number, but an atomic mass or mass number different from the atomic mass or mass number usually found in nature.

Examples of isotopes suitable for inclusion in the compounds of the invention include isotopes of hydrogen, such as $^{2}H$ and $^{3}H$, carbon, such as $^{11}C$, $^{13}C$ and $^{14}C$, chlorine, such as $^{36}Cl$, fluorine, such as $^{18}F$, iodine, such as $^{123}I$ and $^{125}I$, nitrogen, such as $^{13}N$ and $^{15}N$, oxygen, such as $^{15}O$, $^{17}O$ and $^{18}O$, phosphorus, such as $^{32}P$, and sulphur, such as $^{35}S$.

Certain isotopically-labeled compounds of formula (I), for example, those incorporating a radioactive isotope, are useful in drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection.

Substitution with heavier isotopes such as deuterium, i.e. $^{2}H$, may afford certain therapeutic advantages resulting from greater metabolic stability, for example, increased in vivo half-life or reduced dosage requirements, and

hence may be preferred in some circumstances.

Substitution with positron emitting isotopes, such as $^{11}C$, $^{18}F$, $^{15}O$ and $^{13}N$, can be useful in Positron Emission Topography (PET) studies for examining substrate receptor occupancy.

Isotopically-labeled compounds of formula (I) can generally be prepared by conventional techniques known to those skilled in the art or by processes analogous to those described in the accompanying examples and preparations using an appropriate isotopically-labeled reagents in place of the non-labeled reagent previously employed.

All of the compounds of the formula (I) can be prepared by the procedures described in the general methods presented below or by the specific methods described in the examples section and the preparations section, or by routine modifications thereof. The present invention also encompasses any one or more of these processes for preparing the compounds of formula (I), in addition to any novel intermediates used therein.

## General Synthesis

The compounds of the present invention may be prepared by a variety of processes well known for the preparation of compounds of this type, for example as shown in the following Method A.

Unless otherwise indicated, $R^1$, $R^2$, $R^3$, $R^4$, $R^5$, $R^6$, $R^7$, $R^8$, A and B in the following methods are as defined above. All starting materials in the following general syntheses may be commercially available or obtained by conventional methods known to those skilled in the art, such as WO99/55706 and WO 02/20523 and the disclosures of which are incorporated herein by references.

## Method A

This illustrates the preparation of compounds of formula (I).

*Reaction Scheme A*

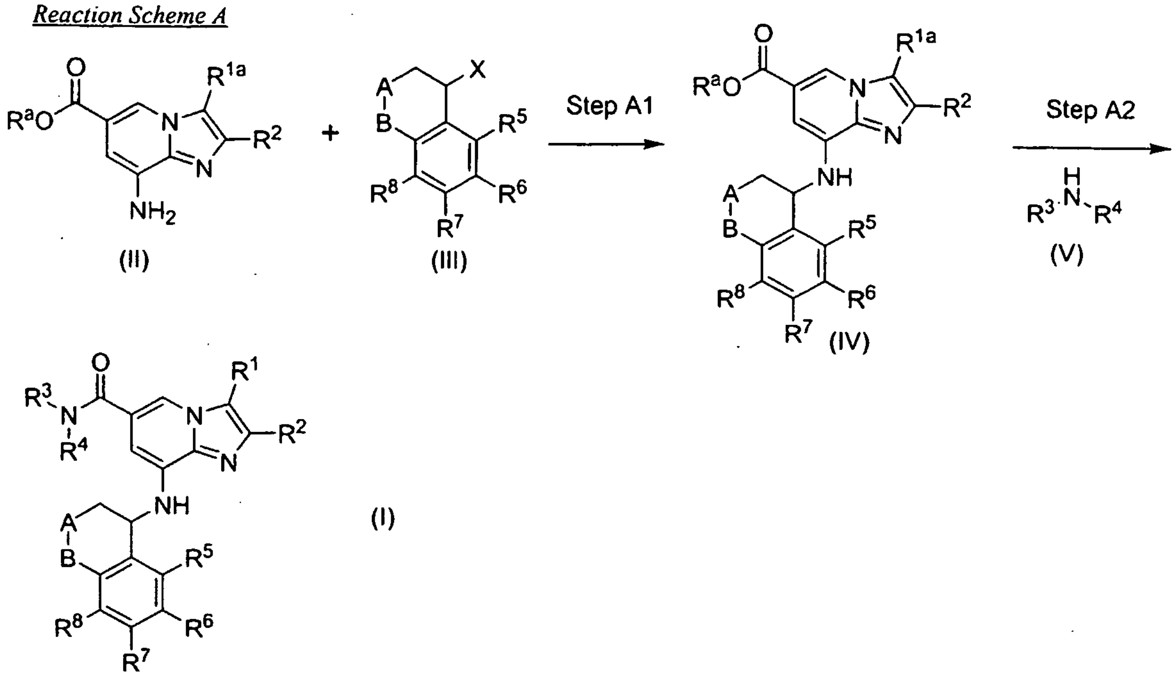


(II) (III) (IV) (V) (I)

In Reaction Scheme A, $R^a$ is a carboxy-protecting group; $R^{1a}$ is $R^1$ as defined above or $R^1$ wherein hydroxy group is protected by a hydroxy-protecting group; and X is a leaving group.

The term "carboxy-protecting group", as used herein, signifies a protecting group capable of being cleaved by various means to yield a carboxy group, such as for example, a $C_1$-$C_6$ alkyl group, halo $C_1$-$C_6$ alkyl group or aryl $C_1$-$C_6$ alkyl group. Of these, a $C_1$-$C_6$ alkyl group and an aryl $C_1$-$C_6$ alkyl group are preferred.

The term "hydroxy-protecting groups", as used herein, signifies a protecting group capable of being cleaved by various means to yield a hydroxy group, such as hydrogenolysis, hydrolysis, electrolysis or photolysis, and such hydroxy-protecting groups are described in Protective Groups in Organic Synthesis edited by T. W. Greene et al. (John Wiley & Sons, 1999). Such as for example, $C_1$-$C_4$ alkoxycarbonyl, $C_1$-$C_4$ alkylcarbonyl, tri-$C_1$-$C_4$ alkylsilyl or tri-$C_1$-$C_4$ alkylarylsilyl groups, and $C_1$-$C_4$ alkoxy-$C_1$-$C_4$ alkyl groups. Suitable hydroxy-protecting groups include acetyl and *tert*-butyldimethylsilyl.

The term "leaving group", as used herein, signifies a group capable of being substituted by nucleophilic groups, such as a hydroxy group, amines or carboanions and examples of such leaving groups include halogen atoms, a alkylsulfonyl group and a phenylsulfonyl group. Of these, a bromine, a chlorine atom, a methylsulfonyl group, a trifluoromethylsulfonyl group and a 4-methylphenylsulfonyl group are preferred.

**Step A1**

In this step, the compound of formula (IV) is prepared by nucleophilic substitution of the compound of formula (III), which is commercially available or may be prepared by the method as described in WO00/078751, with the compound of formula (II), which is commercially available or may be prepared by the methods as described in WO99/55706 and WO02/020523.

The reaction is normally and preferably effected in the presence of solvent. There is no particular restriction on the nature of the solvent to be employed, provided that it has no adverse effect on the reaction or the reagents involved and that it can dissolve reagents, at least to some extent. Examples of suitable solvents include: ethers, such as tetrahydrofuran (THF), ethylene glycol dimethyl ether and dioxane; amides, such as *N,N*-dimethylformamide (DMF), *N,N*-dimethylacetamide (DMA) and *N*-methyl-2-pyrrolidinone (NMP); nitriles, such as acetonitrile; and ketones, such as acetone; alcohols, such as 2-methyl-2-propanol, 1-butanol, 1-propanol, 2-propanol, ethanol and methanol; and sulfoxide, such as dimethyl sulfoxide (DMSO). Of these solvents, amides, ketones and alcohols are preferred. Acetone is more preferred.

The reaction may be carried out with or without a base. There is likewise no particular restriction on the nature of the bases used, and any base commonly used in reactions of this type may equally be used here. Examples of such bases include: alkali metal alkoxides, such as sodium methoxide, sodium ethoxide and potassium *tert*-butoxide; alkali metal carbonates, such as lithium carbonate, sodium carbonate, cesium carbonate, and potassium carbonate; alkali metal hydrogencarbonates, such as sodium hydrogencarbonate and potassium hydrogencarbonate; and organic amines, such as triethylamine, tripropylamine, tributylamine, dicyclohexylamine, *N,N*-diisopropylethylamine, *N*-methylpiperidine, N-methylmorpholine, 1,8-diazabicyclo[5.4.0]undec-7-ene (DBU) and 1,5-diazabicyclo[4.3.0]non-5-ene (DBN). Of these, potassium carbonate is preferred.

The reaction may be carried out with or without an iodide. Examples of such iodides include: sodium iodide, potassium iodide and cesium iodide. Of these, sodium iodide and potassium iodide are preferred.

The reaction can take place over a wide range of temperatures, and the precise reaction temperature is not critical to the invention. The preferred reaction temperature will depend upon such factors as the nature of the solvent, and the starting materials. However, in general, it is convenient to carry out the reaction at a temperature of from about 0˚C to about 250˚C. The time required for the reaction may also vary widely, depending on many factors, notably the reaction temperature and the nature of the starting materials and solvent employed. However, provided that the reaction is effected under the preferred conditions outlined above, a period of from about 5 minutes to about 72 hours will usually suffice.

**Step A2**

In this step, the desired compound of formula (I) is prepared by (A2a1) hydrolysis of the compound of formula (IV) prepared as described in Step A1 followed by (A2a2) condensing reaction with the compound of formula (IV) or (A2b) substituting reaction of the compound of formula (IV) with the compound of formula (V).

(A2a1) hydrolysis

The reaction is normally and preferably effected in the presence of solvent. There is no particular restriction on the nature of the solvent to be employed, provided that it has no adverse effect on the reaction or the reagents involved and that it can dissolve reagents, at least to some extent. Examples of suitable solvents include: ether, such as tetrahydrofuran and dioxane; amides, such as *N,N*-dimethylformamide; alcohols, such as ethanol and methanol; and water. Of these solvents, methanol, tetrahydrofuran and water are preferred.

The reaction is carried out in the presence of a base. There is likewise no particular restriction on the nature of the bases used, and any base commonly used in reactions of this type may equally be used here. Examples of such bases include: alkali metal hydroxides, such as lithium hydroxide, sodium hydroxide and potassium hydroxide. Of these, sodium hydroxide is preferred.

The reaction can take place over a wide range of temperatures, and the precise reaction temperature is not critical to the invention. The preferred reaction temperature will depend upon such factors as the nature of the solvent, and the starting materials. However, in general, it is convenient to carry out the reaction at a temperature of from about 0˚C to about 100˚C. The time required for the reaction may also vary widely, depending on many factors, notably the

reaction temperature and the nature of the starting materials and solvent employed. However, provided that the reaction is effected under the preferred conditions outlined above, a period of from about 5 minutes to about 12 hours will usually suffice.

(A2a2) condensing reaction

The reaction is normally and preferably effected in the presence of solvent. There is no particular restriction on the nature of the solvent to be employed, provided that it has no adverse effect on the reaction or the reagents involved and that it can dissolve reagents, at least to some extent. Examples of suitable solvents include: halogenated hydrocarbons, such as dichloromethane, chloroform, and 1,2-dichloroethane; ethers, such as tetrahydrofuran and dioxane; amides, such as *N,N*-dimethylformamide and *N,N*-dimethylacetamide; and nitriles, such as acetonitrile. Of these solvents, halogenated hydrocarbons and amides are preferred; dichloromethane and *N,N*-dimethylformamide are more preferred.

The reaction is carried out in the presence of a condensing agent. There is likewise no particular restriction on the nature of the condensing agents used, and any condensing agent commonly used in reactions of this type may equally be used here. Examples of such condensing agents include: azodicarboxylic acid di-lower alkyl ester- triphenylphosphines, such as diethyl azodicarboxylate-triphenylphosphine; 2-halo-1-lower alkyl pyridinium halides, such as 2-chloro-1-methy pyridinium iodide and 2-bromo-1-ethylpyridinium tetrafluoroborate (BEP); diarylphosphorylazides, such as diphenylphosphorylazide (DPPA); chloroformates, such as ethyl chloroformate and isobutyl chloroformate; phosphorocyanidates, such as diethyl phosphorocyanidate (DEPC); imidazole derivatives, such as *N,N'*-carbonyldiimidazole (CDI); carbodiimide derivatives, such as *N,N'*-dicyclohexylcarbodiimide (DCC) and 1-(3- dimethylaminopropyl)-3-ethylcarbodiimide hydrochloride (EDCI); iminium salts, such as 2-(1*H*-benzotriazol-1-yl)-1,1,3,3-tetramethyluronium hexafluorophosphate (HBTU) and tetramethyl fluoroformamidinium hexafluoro phosphate (TFFH); and phosphonium salts, such as benzotriazol-1-yloxytris(dimethylamino)phosphonium hexafluorophosphate (BOP) and bromo-tris-pyrrolidino-phosphonium hexafluorophosphate (PyBrop). Of these, EDCI is preferred.

Reagents, such as 4-(*N,N*-dimethylamino)pyridine (DMAP), and *N*-hydroxybenztriazole (HOBt), may be employed for this step. Of these, HOBt is preferred.

The reaction may be carried out with or without a base. There is likewise no particular restriction on the nature of the bases used, and any base commonly used in reactions of this type may equally be used here. Examples of such bases include: amines, such as *N*-methylmorpholine, triethylamine, diisopropylethylamine, *N*-methylpiperidine and pyridine. Of these, triethylamine and *N*-methylmorpholine are preferred.

The reaction can take place over a wide range of temperatures, and the precise reaction temperature is not critical to the invention. The preferred reaction temperature will depend upon such factors as the nature of the solvent, and the starting materials. However, in general, it is convenient to carry out the reaction at a temperature of from about 0˚C to about 80˚C. The time required for the reaction may also vary widely, depending on many factors, notably the reaction temperature and the nature of the starting materials and solvent employed. However, provided that the reaction is effected under the preferred conditions outlined above, a period of from about 5 minutes to about 24 hours will usually suffice. (A2b) substituting reaction

The reaction can be carried out by heating the reactants in the neat amino compound or in an inert solvent under standard condition. There is no particular restriction on the nature of the solvent to be employed, provided that it has no adverse effect on the reaction or the reagents involved and that it can dissolve reagents, at least to some extent. Examples of suitable solvents include: ethers, such as ethylene glycol dimethyl ether, tetrahydrofuran and dioxane; amides, such as *N,N*-dimethylformamide and *N,N*-dimethylacetamide; nitriles, such as acetonitrile; and alcohols such as 2-methyl-2-propanol, 1-butanol, 1-propanol, 2-propanol, ethanol and methanol. Of these solvents, ethers and alcohols are preferred. Tetrahydrofuran is more preferred.

The reaction may be carried out with or without a catalyst. There is likewise no particular restriction on the nature of the catalysts used, and any catalysts commonly used in reactions of this type may equally be used here. Examples of such catalysts include: sodium cyanide or potassium cyanide. Of these, sodium cyanide is preferred.

The reaction can take place over a wide range of temperatures, and the precise reaction temperature is not critical to the invention. The preferred reaction temperature will depend upon such factors as the nature of the solvent, and the starting materials. However, in general, it is convenient to carry out the reaction at a temperature of from about 40˚C to about 200˚C. The time required for the reaction may also vary widely, depending on many factors, notably the reaction temperature and the nature of the starting materials and solvent employed. However, provided that the reaction is effected under the preferred conditions outlined above, a period of from about 30 minutes to about 24 hours will usually suffice.

Introduction of the hydroxy-protecting group

In the case where $R^1$ has a hydroxy group, if necessary, the reaction may be accomplished after protecting the hydroxy group, before the reaction affected by the hydroxy group.
The introduction of the hydroxy-protecting group can be carried out at an appropriate step.
This reaction is described in detail by T. W. Greene et al., Protective Groups in Organic Synthesis, 369-453, (1999), the disclosures of which are incorporated herein by reference. The following exemplifies a typical reaction involving the protecting group *tert*-butyldimetylsilyl.
For example, when the hydroxy-protecting group is a " tert-butyldimetylsilyl ", this step is conducted by reacting with a desired hydroxy- protecting group halide in an inert solvent in the presence of a base.
Examples of suitable solvents include: halogenated hydrocarbons, such as dichloromethane, chloroform, carbon tetrachloride and 1,2-dichloroethane; ethers, such as diethyl ether, diisopropyl ether, tetrahydrofuran and dioxane; aromatic hydrocarbons, such as benzene, toluene and nitrobenzene; amides, such as formamide, *N,N*-dimethylformamide, *N,N*-dimethylacetamide and hexamethylphosphoric triamide; or mixed solvents thereof. Of these, tetrahydrofuran or *N,N*-dimethylformamide is preferred.
Examples of the hydroxy-protecting group halide usable in the above reaction include trimethylsilyl chloride, triethylsilyl chloride, *tert*-butyldimethylsilyl chloride, *tert*-butyldimethylsilyl bromide, acetyl chloride are preferred.
Examples of the base include alkali metal hydroxides such as lithium hydroxide, sodium hydroxide and potassium hydroxide, alkali metal carbonates such as lithium carbonate, sodium carbonate and potassium carbonate, and organic amines such as triethylamine, tributylamine, *N*- methylmorpholine, pyridine, imidazole, 4-dimethylaminopyridine, picoline, lutidine, collidine, DBN and DBU. Out of these, triethylamine, imidazole, or pyridine is preferred. Upon use of an organic amine in the liquid form, it also serves as a solvent when used in large excess.
Although the reaction temperature differs with the nature of the starting compound, the halide and the solvent, it usually ranges from 0˚C to 80˚C (preferably 0 to 30˚C). Although the reaction time differs with the reaction temperature or the like, it ranges from 10 minutes to 2 days (preferably 30 minutes to 1 day).

Deprotecting step

In the case where $R^{1a}$ has a protected hydroxy group, the deprotection reaction will follow to yield a hydroxy group. This reaction is described in detail by T. W. Greene et al., Protective Groups in Organic Synthesis, 369-453, (1999), the disclosures of which are incorporated herein by reference. The following exemplifies a typical reaction involving the protecting group *tert*-butyldimetylsilyl.
The deprotection of the hydroxyl groups is carried out with an acid, such as acetic acid, hydrogen fluoride, hydrogen fluoride-pyridine complex, or fluoride ion, such as tetrabutylammonium fluoride (TBAF).
The deprotection reaction is normally and preferably effected in the presence of solvent. There is no particular restriction on the nature of the solvent to be employed, provided that it has no adverse effect on the reaction or the reagents involved and that it can dissolve reagents, at least to some extent. Examples of suitable solvents include, but are not limited to: alcohol, such as methanol, ethanol or mixed solvents thereof.
The deprotection reaction can take place over a wide range of temperatures, and the precise reaction temperature is not critical to the invention. The preferred reaction temperature will depend upon such factors as the nature of the solvent, and the starting materials. However, in general, it is convenient to carry out the reaction at a temperature of from about 0˚C to about 100˚C. The time required for the reaction may also vary widely, depending on many factors, notably the reaction temperature and the nature of the starting materials and solvent employed. However, provided that the reaction is effected under the preferred conditions outlined above, a period of from about 10 minutes to about 24 hours, will usually suffice.
The compounds of formula (I), and the intermediates in the above-mentioned preparation methods can be isolated and purified by conventional procedures, such as distillation, recrystallization or chromatographic purification.
Compounds of the invention intended for pharmaceutical use may be administered as crystalline or amorphous products. They may be obtained, for example, as solid plugs, powders, or films by methods such as precipitation, crystallization, freeze-drying, spray drying, or evaporative drying. Microwave or radio frequency drying may be used for this purpose.
Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC).
Alternatively, a method of optical resolution of a racemate (or a racemic precursor) can be appropriately selected from conventional procedures, for example, preferential crystallization, or resolution of diastereomeric salts between a basic moiety of the compound of formula (I) and a suitable optically active acid such as tartaric acid.
Compounds of the invention intended for pharmaceutical use may be administered as crystalline or amorphous

products. They may be obtained, for example, as solid plugs, powders, or films by methods such as precipitation, crystallization, freeze-drying, spray drying, or evaporative drying. Microwave or radio frequency drying may be used for this purpose.

They may be administered alone or in combination with one or more other compounds of the invention or in combination with one or more other drugs (or as any combination thereof). Generally, they will be administered as a pharmaceutical composition or formulation in association with one or more pharmaceutically acceptable carriers or excipients. The term "carrier" or "excipient" is used herein to describe any ingredient other than the compound(s) of the invention. The choice of carrier or excipient will to a large extent depend on factors such as the particular mode of administration, the effect of the excipient on solubility and stability, and the nature of the dosage form.

Pharmaceutical compositions suitable for the delivery of compounds of the present invention and methods for their preparation will be readily apparent to those skilled in the art. Such compositions and methods for their preparation may be found, for example, in 'Remington's Pharmaceutical Sciences', 19th Edition (Mack Publishing Company, 1995).

ORAL ADMINISTRATION

The compounds of the invention may be administered orally. Oral administration may involve swallowing, so that the compound enters the gastrointestinal tract, or buccal or sublingual administration may be employed by which the compound enters the blood stream directly from the mouth.

Formulations suitable for oral administration include solid formulations such as, for example, tablets, capsules containing particulates, liquids, or powders, lozenges (including liquid-filled), chews, multi- and nano-particulates, gels, solid solution, liposome, films (including muco-adhesive), ovules, sprays and liquid formulations.

Liquid formulations include, for example, suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986 by Liang and Chen (2001).

For tablet dosage forms, depending on dose, the drug may make up from about 1 wt% to about 80 wt% of the dosage form, more typically from about 5 wt% to about 60 wt% of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkyl-substituted hydroxypropyl cellulose, starch, pregelatinised starch and sodium alginate. Generally, the disintegrant will comprise from about 1 wt% to about 25 wt%, preferably from about 5 wt% to about 20 wt% of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface-active agents, such as sodium lauryl sulfate and polysorbate 80, and glidants such as silicon dioxide and talc. When present, surface active agents may comprise from about 0.2 wt% to about 5 wt% of the tablet, and glidants may comprise from about 0.2 wt% to about 1 wt% of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from about 0.25 wt% to about 10 wt%, preferably from about 0.5 wt% to about 3 wt% of the tablet.

Other possible ingredients include anti-oxidants, colourants, flavouring agents, preservatives and taste-masking agents.

Exemplary tablets contain up to about 80% drug, from about 10 wt% to about 90 wt% binder, from about 0 wt% to about 85 wt% diluent, from about 2 wt% to about 10 wt% disintegrant, and from about 0.25 wt% to about 10 wt% lubricant.

Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated.

The formulation of tablets is discussed in "Pharmaceutical Dosage Forms: Tablets, Vol. 1", by H. Lieberman and L. Lachman, Marcel Dekker, N.Y., N.Y., 1980 (ISBN 0-8247-6918-X).

Solid formulations for oral administration may be formulated to be immediate and/or modified release. Modified release formulations include delayed-, sustained-, pulsed-, controlled-, targeted and programmed release.

Suitable modified release formulations for the purposes of the invention are described in US Patent No. 6,106,864. Details of other suitable release technologies such as high energy dispersions and osmotic and coated

particles are to be found in Verma et al, Pharmaceutical Technology On-line, 25(2), 1-14 (2001). The use of chewing gum to achieve controlled release is described in WO00/35298.

PARENTERAL ADMINISTRATION

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from about 3 to about 9), but, for some applications, they may be more suitably formulated as a sterile non-aqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula (I) used in the preparation of parenteral solutions may be increased by the use of appropriate formulation techniques, such as the incorporation of solubility-enhancing agents.

Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and PGLA microspheres.

TOPICAL ADMINISTRATION

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958 by Finnin and Morgan (October 1999).

Other means of topical administration include delivery by electroporation, iontophoresis, phonophoresis, sonophoresis and microneedle or needle-free (*e.g.* Powderject™, Bioject™, etc.) injection.

Formulations for topical administration may be formulated to be immediate and/or modified release. Modified release formulations include delayed-, sustained-, pulsed-, controlled-, targeted and programmed release.

INHALED/INTRANASAL ADMINISTRATION

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurized container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurized container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, supercritical fluid processing to form nanoparticles, high pressure homogenization, or spray drying.

Capsules (made, for example, from gelatin or HPMC), blisters and cartridges for use in an inhaler or insufflator may be formulated to contain a powder mix of the compound of the invention, a suitable powder base such as lactose or starch and a performance modifier such as /-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from about 1μg to about 20mg of the compound of the invention per actuation and the actuation volume may vary from about 1μl to about 100μl. A typical formulation may comprise a compound of formula (I), propylene glycol, sterile water, ethanol and sodium chloride. Alternative solvents which may be used instead of propylene glycol include glycerol and polyethylene glycol.

Suitable flavors, such as menthol and levomenthol, or sweeteners, such as saccharin or saccharin sodium, may be added to those formulations of the invention intended for inhaled/intranasal administration. Formulations for inhaled/intranasal administration may be formulated to be immediate and/or modified release using, for example, poly (DL-lactic-coglycolic acid (PGLA). Modified release formulations include delayed-, sustained-, pulsed-, controlled-, targeted and programmed release.

In the case of dry powder inhalers and aerosols, the dosage unit is determined by means of a valve which delivers a metered amount. Units in accordance with the invention are typically arranged to administer a metered dose or "puff' containing from about 1 to about 100μg of the compound of formula (I). The overall daily dose will typically be in the range about 50 μg to about 20 mg which may be administered in a single dose or, more usually, as divided doses throughout the day.

RECTAL/INTRAVAGINAL ADMINISTRATION

The compounds of the invention may be administered rectally or vaginally, for example, in the form of a suppository, pessary, or enema. Cocoa butter is a traditional suppository base, but various alternatives may be used as appropriate.

Formulations for rectal/vaginal administration may be formulated to be immediate and/or modified release. Modified release formulations include delayed-, sustained-, pulsed-, controlled-, targeted and programmed release.

OCULAR/AURAL ADMINISTRATION

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (*e.g*. absorbable gel sponges, collagen) and non-biodegradable (*e.g*. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

Formulations for ocular/aural administration may be formulated to be immediate and/or modified release. Modified release formulations include delayed-, sustained-, pulsed-, controlled-, targeted, or programmed release.

OTHER TECHNOLOGIES

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, *i.e*. as a carrier, diluent, or solubiliser. Most commonly used for these purposes are alpha-, beta- and gamma-cyclodextrins, examples of which may be found in. WO91/11172, WO94/02518 and WO98/55148.

KIT-OF-PARTS

Inasmuch as it may be desirable to administer a combination of active compounds, for example, for the purpose of treating a particular disease or condition, it is within the scope of the present invention that two or more pharmaceutical compositions, at least one of which contains a compound in accordance with the invention, may conveniently be combined in the form of a kit suitable for coadministration of the compositions.

Thus the kit of the invention comprises two or more separate pharmaceutical compositions, at least one of which contains a compound of formula (I) in accordance with the invention, and means for separately retaining said compositions, such as a container, divided bottle, or divided foil packet. An example of such a kit is the familiar blister pack used for the packaging of tablets, capsules and the like.

The kit of the invention is particularly suitable for administering different dosage forms, for example, oral and parenteral, for administering the separate compositions at different dosage intervals, or for titrating the separate compositions against one another. To assist compliance, the kit typically comprises directions for administration and may be provided with a so-called memory aid.

DOSAGE

For administration to human patients, the total daily dose of the compounds of the invention is typically in the range of about 0.05 mg to about 500 mg depending, of course, on the mode of administration, preferred in the range of about 0.1 mg to about 400 mg and more preferred in the range of about 0.5 mg to about 300 mg. For example, oral administration may require a total daily dose of from about 1 mg to about 300 mg, while an intravenous dose may only require from about 0.5 mg to about 100 mg. The total daily dose may be administered in single or divided doses.

These dosages are based on an average human subject having a weight of about 65 kg to about 70 kg. The physician will readily be able to determine doses for subjects whose weight falls outside this range, such as infants and the elderly.

COMBINATIONS

As discussed above, a compound of the invention exhibits acid pump inhibitory activity. An acid pump antagonist of the present invention may be usefully combined with another pharmacologically active compound, or with two or more other pharmacologically active compounds, particularly in the treatment of gastroesophageal reflux disease. For example, an acid pump antagonist, particularly a compound of the formula (I), or a pharmaceutically acceptable salt thereof, as defined above, may be administered simultaneously, sequentially or separately in combination with one or more agents selected from:

(i) histamine $H_2$ receptor antagonists, e.g. ranitidine, lafutidine, nizatidine, cimetidine, famotidine and roxatidine;
(ii) proton pump inhibitors, e.g. omeprazole, esomeprazole, pantoprazole, rabeprazole, tenatoprazole, ilaprazole and lansoprazole;
(iii) oral antacid mixtures, e.g. Maalox®, Aludrox® and Gaviscon®;
(iv) mucosal protective agents, e.g. polaprezinc, ecabet sodium, rebamipide, teprenone, cetraxate, sucralfate, chloropylline-copper and plaunotol;
(v) anti-gastric agents, e.g. Anti-gastrin vaccine, itriglumide and Z-360;
(vi) 5-$HT_3$ antagonists, e.g. dolasetron, palonosetron, alosetron, azasetron, ramosetron, mitrazapine, granisetron, tropisetron, E-3620, ondansetron and indisetron;
(vii) 5-$HT_4$ agonists, e.g. tegaserod, mosapride, cinitapride and oxtriptane;
(viii) laxatives, e.g. Trifyba®, Fybogel®, Konsyl®, Isogel®, Regulan®, Celevac® and Normacol®;
(ix) $GABA_B$ agonists, e.g. baclofen and AZD-3355;
(x) $GABA_B$ antagonists, e.g. GAS-360 and SGS-742;
(xi) calcium channel blockers, e.g. aranidipine, lacidipine, falodipine, azelnidipine, clinidipine, lomerizine, diltiazem, gallopamil, efonidipine, nisoldipine, amlodipine, lercanidipine, bevantolol, nicardipine, isradipine, benidipine, verapamil, nitrendipine, barnidipine, propafenone, manidipine, bepridil, nifedipine, nilvadipine, nimodipine and fasudil;
(xii) dopamine antagonists, e.g. metoclopramide, domperidone and levosulpiride;
(xiii) Tachykinin (NK) antagonists, particularly NK-3, NK-2 and NK-1 antagonists, e.g. nepadutant, saredutant, talnetant, (αR,9R)-7-[3,5-bis(trifluoromethyl)benzyl]-8,9,10,11-tetrahydro-9-methyl-5-(4-methylphenyl)-7H-[1,4]diazocino[2,1-g][1,7]naphthridine-6-13-dione (TAK-637), 5-[[(2R,3S)-2-[(1R)-1-[3,5-bis(trifluoromethyl)phenyl]ethoxy-3-(4-fluorophenyl)-4-morpholinyl]methyl]-1,2-dihydro-3 H-1,2,4-triazol-3-one (MK-869), lanepitant, dapitant and 3-[[2-methoxy-5-(trifluoromethoxy)phenyl] methylamino]-2-phenyl-piperidine (2S,3S);
(xiv) Helicobacter pylori infection agents, e.g. clarithromicyn, roxithromycin, rokitamycin, flurithromycin, telithromycin, amoxicillin, ampicillin, temocillin, bacampicillin, aspoxicillin, sultamicillin, piperacillin, lenampicillin, tetracycline, metronidazole, bithmuth citrate and bithmuth subsalicylate;
(xv) nitric oxide synthase inhibitors, e.g. GW-274150, tilarginine, P54, guanidioethyldisulfide and nitroflurbiprofen;
(xvi) vanilloid receptor 1 antagonists, e.g. AMG-517 and GW-705498;
(xvii) muscarinic receptor antagonists, e.g. trospium, solifenacin, tolterodine, tiotropium, cimetropium, oxitropium, ipratropium, tiquizium, dalifenacin and imidafenacin;
(xviii) calmodulin antagonists, e.g. squalamine and DY-9760;
(xix) potassium channel agonists, e.g. pinacidil, tilisolol, nicorandil, NS-8 and retigabine;
(xx) beta-1 agonists, e.g. dobutamine, denopamine, xamoterol, denopamine, docarpamine and xamoterol;
(xxi) beta-2 agonists, e.g. salbutamol; terbutaline, arformoterol, meluadrine, mabuterol, ritodrine, fenoterol, clenbuterol, formoterol, procaterol, tulobuterol, pirbuterol, bambuterol, tulobuterol, dopexamine and levosalbutamol;
(xxii) beta agonists, e.g. isoproterenol and terbutaline;
(xxiii) alpha 2 agonists, e.g. clonidine, medetomidine, lofexidine, moxonidine, tizanidine, guanfacine, guanabenz, talipexole and dexmedetomidine;
(xxiv) endthelin A antagonists, e.g. bonsetan, atrasentan, ambrisentan, clazosentan, sitaxsentan, fandosentan and darusentan;
(xxv) opioid μ agonists, e.g. morphine, fentanyl and loperamide;
(xxvi) opioid μ antagonists, e.g. naloxone, buprenorphine and alvimopan;
(xxvii) motilin agonists, e.g. erythromycin, mitemcinal, SLV-305 and atilmotin;
(xxviii) ghrelin agonists, e.g. capromorelin and TZP-101;
(xxix) AchE release stimulants, e.g. Z-338 and KW-5092;
(xxx) CCK-B antagonists, e.g. itriglumide, YF-476 and S-0509;
(xxxi) glucagon antagonists, e.g. NN-2501 and A-770077;
(xxxii) piperacillin, lenampicillin, tetracycline, metronidazole, bithmuth citrate and bithmuth subsalicylate;
(xxxiii) Glucagon-like peptide-1 (GLP-1) antagonists, e.g. PNU-126814;
(xxxiv) small conductance calcium-activated potassium channel 3 (SK-3) antagonists, e.g. apamin, dequalinium, atracurium, pancuronium and tubocurarine.

**Method for assessing biological activities:**

The acid pump inhibitory activity and other biological activities of the compounds of this invention were determined by the following procedures. Symbols have their usual meanings: mL (milliliter(s)), μL (microlitter(s)), Kg (kirogram (s)), g (gram(s)), mg (milligram(s)), μg (microgram(s)), pmol (pico molar(s)), mmol (milli molar(s)), M (molar mass ($m^3$/mol)), mM (milli molar mass), (μM (micro molar mass), quant. (quantitative yield), nm (nanometer(s)), min (minute (s)) Cat# (catalog number).

**Preparation of gastric vesicles from fresh porcine stomachs**

The porcine gastric vesicles for Porcine gastric $H^+/K^+$-ATPase inhibition assays were prepared from mucous membrane in fresh porcine stomachs by homogenization with a tight-fitted polytetrafluoroethylene (Teflone®) homogenizer in 0.25 M sucrose at 4°C. The crude pellet was removed with centrifugation at 20,000 g for 30 min. Then supernatant was centrifuged at 100,000 g for 30 min. The resulting pellet was re-suspended in 0.25 M sucrose, and then subjected to density gradient centrifugation at 132,000 g for 90 min. The gastric vesicles were collected from interface on 0.25 M sucrose layer containing 7% Ficoll™ PM400(Amersham Biosciences). This procedure was performed in a cold room.

**Ion-leaky Porcine gastric H+/K+-ATPase inhibition**

Ion-leaky porcine gastric $H^+/K^+$-ATPase_inhibition was measured according to the modified method described in Biochemical Pharmacology, 1988, 37, 2231-2236.
The isolated vesicles were lyophilized, and then kept in deep-freezer until use. For enzyme assay, lyophilized vesicles were reconstituted with 3 mM $MgSO_4$ containing 40 mM Bis-tris (pH 6.4 at 37°C).
Enzyme reaction was performed incubating 5 mM KCl, 3 mM $Na_2ATP$, 3 mM $MgSO_4$ and 1.0 μg of reconstituted vesicles for 30 minutes at 37°C in a final 60 μl of reaction mixture (40 mM Bis-tris, pH 6.4) with or without the test compound. Enzyme reaction was stopped by adding 10% sodium dodecyl sulphate (SDS). Released inorganic phosphate from ATP was detected by incubation with mixture of 1 part of 35 mM ammonium molybdate tetrahydrate in 15 mM Zinc acetate hydrate and 4 parts of 10% ascorbic acid (pH 5.0), resulting in phosphomolybdate, which has optical density at 750 nm. All example compounds showed potent inhibitory activity.
The results of $IC_{50}$ values of the inhibitory activity for the compounds of following examples are shown in Table 1.

Table 1.

| Example No. | $IC_{50}$ (μM) |
|---|---|
| 1-1 | 0.085 |
| 1-2 | 0.0619 |
| 1-3 | 0.0278 |
| 2-1 | 0.0497 |

(continued)

| Example No. | $IC_{50}$ (μM) |
|---|---|
| 2-2 | 0.0811 |
| 2-3 | 0.064 |
| 3-1 | 0.103 |
| 3-2 | 0.125 |
| 3-3 | 0.0676 |
| 4-1 | 0.0871 |
| 4-2 | 0.273 |
| 4-3 | 0.112 |
| 5-1 | 0.0947 |
| 5-2 | 0.0862 |
| 5-3 | 0.093 |
| 6-1 | 0.0139 |
| 6-2 | 0.0409 |
| 6-3 | 0.0157 |
| 7-1 | 0.0266 |
| 7-2 | 0.0205 |
| 7-3 | 0.0559 |
| 8-1 | 0.02 |
| 8-2 | 0.023 |
| 8-3 | 0.049 |
| 9-1 | 0.05 |
| 9-2 | 0.0159 |
| 9-3 | 0.0532 |
| 10-1 | 0.0751 |
| 10-2 | 0.0511 |
| 10-3 | 0.091 |
| 11-1 | 0.0486 |
| 11-2 | 0.0538 |
| 11-3 | 0.101 |
| 12-1 | 0.0208 |
| 12-2 | 0.197 |
| 12-3 | 0.0811 |
| 13-1 | 0.25 |
| 13-2 | 1.1 |
| 13-3 | 0.17 |
| 14-1 | 0.294 |
| 14-2 | 0.12 |
| 14-3 | 0.17 |

(continued)

| Example No. | $IC_{50}$ (μM) |
|---|---|
| 15-1 | 0.0409 |
| 15-2 | 0.23 |
| 15-3 | 0.37 |
| 16-1 | 0.331 |
| 16-2 | 0.1 |
| 16-3 | 0.18 |
| 17-1 | 0.064 |
| 17-2 | 0.043 |
| 17-3 | 0.091 |
| 18-1 | 0.35 |
| 18-2 | 0.3 |
| 18-3 | 0.24 |
| 19-1 | 0.39 |
| 19-2 | 0.6 |
| 19-3 | 0.5 |

**Ion-tight porcine gastric $H^+/K^+$-ATPase inhibition**

Ion-tight porcine gastric $H^+/K^+$-ATPase inhibition was measured according to the modified method described in Biochemical Pharmacology, 1988, 37, 2231-2236.

The isolated vesicles were kept in deep-freezer until use. For enzyme assay, vesicles were diluted with 3 mM $MgSO_4$ containing 5 mM Tris (pH 7.4 at 37˚C).

Enzyme reaction was performed incubating 150 mM KCl, 3 mM $Na_2ATP$, 3 mM $MgSO_4$, 15 μM valinomycin and 3.0 μg of vesicles for 30 minutes at 37˚C in a final 60 μl of reaction mixture (5mM Tris, pH 7.4) with or without the test compound. Enzyme reaction was stopped by adding 10% SDS. Released inorganic phosphate from ATP was detected by incubating with mixture of 1 part of 35 mM ammonium molybdate tetrahydrate in 15 mM Zinc acetate hydrate and 4 parts of 10% ascorbic acid (pH 5.0), resulting in phosphomolybdate, which has optical density at 750 nm.

**Canine kidney $Na^+/K^+$-ATPase inhibition**

The powdered canine kidney $Na^+/K^+$-ATPase (Sigma) was reconstituted with 3 mM $MgSO_4$ containing 40 mM Tris (pH 7.4 at 37˚C). Enzyme reaction was performed incubating 100 mM NaCl, 2 mM KCl, 3 mM $Na_2ATP$, 3 mM $MgSO_4$ and 12 μg of enzyme for 30 minutes at 37˚C in a final 60 μl of reaction mixture (40 mM Tris, pH 7.4) with or without the test compound. Enzyme reaction was stopped by adding 10% SDS. Released inorganic phosphate from ATP was detected by incubating with mixture of 1 part of 35 mM ammonium molybdate tetrahydrate in 15 mM Zinc acetate hydrate and 4 parts of 10% ascorbic acid (pH 5.0), resulting in phosphomolybdate, which has optical density at 750 nm.

**Inhibition of acid secretion in the gastric lumen-perfused rat**

Acid secretion in the gastric lumen-perfused rat was measured according to Watanabe et al. [Watanabe K et al., J. Physiol. (Paris) 2000; 94: 111-116].

Male Sprague-Dawley rats, 8 weeks old, deprived of food for 18 hours before the experiment with free access to water, were anesthetized with urethane (1.4 g/kg, i.p.) and tracheotomized. After a middle abdominal incision, a dual polyethylene cannula was inserted into the forestomach and the stomach was perfused with saline (37 ˚C, pH 5.0) at a rate of 1 ml/min. The acid output in the perfusate was determined at 5 minutes interval by titration with 0.02 M NaOH to pH 5.0. After the determination of basal acid secretion for 30 min, the acid secretion was stimulated by a continuous intravenous infusion of pentagastrin (16 μg/kg/h). The test compounds were administered by an intravenous bolus injection or

intraduodenal administration after the stimulated acid secretion reached a plateau phase. The acid secretion was monitored after the administration.

The activity was evaluated either inhibition of total acid secretion from 0 hours to 1.5 or 3.5 hours after administration or the maximum inhibition after administration.

The compound of Example 5-3 showed a good inhibitory activity.

**Inhibition of gastric acid secretion in the Heidenhain pouch dog**

Male Beagle dogs weighing 7 - 15 kg with Heidenhain pouch [Heidenhain R: Arch Ges Physiol. 1879; 19: 148-167] were used. The animals were allowed to recover from surgery for at least three weeks before the experiments. The animals were kept at a 12 hour light-dark rhythm, housed singly. They received standard food once daily at 11:00 a.m. and tap water ad libitum, and were fasted overnight prior to the experiment, with free access to water. Gastric juice samples were collected throughout the experiment by gravity drainage every 15 min. Acidity in the gastric juice was measured by titration to the end point of pH 7.0. Acid secretion was stimulated by a continuous intravenous infusion of histamine (80 μg/kg/h). Oral or intravenous bolus administration of the test compounds was done 90 minutes after commencement of the histamine infusion. The acid secretion was monitored after the administration. The activity was evaluated by the maximum inhibition relative to the corresponding control value.

**Human dofetilide binding**

Human ether a-go-go related gene (HERG) transfected HEK293S cells were prepared and grown in-house. Cell paste of HEK-293 cells expressing the HERG product can be suspended in 10-fold volume of 50 mM Tris buffer adjusted at pH 7.5 at 25 ˚C with 2 M HCl containing 1 mM $MgCl_2$, 10 mM KCl. The cells were homogenized using a Polytron homogenizer (at the maximum power for 20 seconds) and centrifuged at 48,000 g for 20 minutes at 4˚C. The pellet was resuspended, homogenized and centrifuged once more in the same manner. The resultant supernatant was discarded and the final pellet was resuspended (10-fold volume of 50 mM Tris buffer) and homogenized at the maximum power for 20 seconds. The membrane homogenate was aliquoted and stored at -80˚C until use. An aliquot was used for protein concentration determination using a Protein Assay Rapid Kit (wako) and Spectra max plate reader (Wallac). All the manipulation, stock solution and equipment were kept on ice at all times. For saturation assays, experiments were conducted in a total volume of 200 μl. Saturation was determined by incubating 36 μl of [$^3$H]-dofetilide, and 160 μl of membrane homogenates (20-30 μg protein per well) for 60 minutes at room temperature in the absence or presence of 10μM dofetilide at final concentrations (4 μl) for total or nonspecific binding, respectively. All incubations were terminated by rapid vacuum filtration over PEI soaked glass fiber filter papers using Skatron cell harvester followed by two washes with 50 mM Tris buffer (pH 7.4 at 25 ˚C). Receptor-bound radioactivity was quantified by liquid scintillation counting using Packard LS counter.

For the competition assay, compounds were diluted in 96 well polypropylene plates as 4-point dilutions in semilog format. All dilutions were performed in DMSO first and then transferred into 50 mM Tris buffer (pH 7.4 at 25 ˚C) containing 1 mM $MgCl_2$, 10 mM KCl so that the final DMSO concentration became equal to 1%. Compounds were dispensed in triplicate in assay plates (4 μl). Total binding and nonspecific binding wells were set up in 6 wells as vehicle and 10 μM dofetilide at final concentration, respectively. The radioligand was prepared at 5.6x final concentration and this solution was added to each well (36 μl). The assay was initiated by addition of YSi poly-L-lysine SPA beads (50 μl, 1 mg/well) and membranes (110 μl, 20 μg/well). Incubation was continued for 60 minutes at room temperature. Plates were incubated for a further 3 hours at room temperature for beads to settle. Receptor-bound radioactivity was quantified by counting Wallac MicroBeta plate counter.

**Caco-2 Permeability**

Caco-2 permeability was measured according to the method described in Shiyin Yee, Pharmaceutical Research, 763 (1997).

Caco-2 cells were grown on filter supports (Falcon HTS multiwell insert system) for 14 days. Culture medium was removed from both the apical and basolateral compartments and the monolayers were preincubated with pre-warmed 0.3 ml apical buffer and 1.0 ml basolateral buffer for 0.5 hour at 37˚C in a shaker water bath at 50 cycles/min. The apical buffer consisted of Hanks Balanced Salt Solution, 25 mM D-glucose monohydrate, 20 mM 2-morpholinoethanesulphonic acid (MES) Biological Buffer, 1.25 mM $CaCl_2$ and 0.5 mM $MgCl_2$ (pH 6.5). The basolateral buffer consisted of Hanks Balanced Salt Solution, 25 mM D-glucose monohydrate, 20 mM 2-[4-(2-hydroxyethyl)-l-piperazinyl] ethanesulfonic acid (HEPES) Biological Buffer, 1.25 mM $CaCl_2$ and 0.5 mM $MgCl_2$ (pH 7.4). At the end of the preincubation, the media was removed and test compound solution (10 μM) in buffer was added to the apical compartment. The inserts were moved to wells containing fresh basolateral buffer at 1 hour. Drug concentration in the buffer was

measured by LC/MS analysis.

Flux rate (F, mass/time) was calculated from the slope of cumulative appearance of substrate on the receiver side and apparent permeability coefficient ($P_{app}$) was calculated from the following equation.

$$P_{app} \text{ (cm/sec)} = (F * VD) / (SA * MD)$$

where SA is surface area for transport (0.3 $cm^2$), VD is the donor volume (0.3 ml), MD is the total amount of drug on the donor side at t = 0. All data represent the mean of 2 inserts. Monolayer integrity was determined by Lucifer Yellow transport.

## Half-life in human liver microsomes (HLM)

Test compounds (1 μM) were incubated with 3.3 mM $MgCl_2$ and 0.78 mg/mL HLM (HL101) in 100 mM potassium phosphate buffer (pH 7.4) at 37°C on the 96-deep well plate. The reaction mixture was split into two groups, a non-P450 and a P450 group. NADPH was only added to the reaction mixture of the P450 group. An aliquot of samples of P450 group was collected at 0, 10, 30, and 60 minutes time point, where 0 minutes time point indicated the time when NADPH was added into the reaction mixture of P450 group. An aliquot of samples of non-P450 group was collected at -10 and 65 minutes time point. Collected aliquots were extracted with acetonitrile solution containing an internal standard. The precipitated protein was spun down in centrifuge (2000 rpm, 15 min). The compound concentration in supernatant was measured by LC/MS/MS system.

The half-life value was obtained by plotting the natural logarithm of the peak area ratio of compounds/ internal standard versus time. The slope of the line of best fit through the points yields the rate of metabolism (k). This was converted to a half-life value using following equations:

$$\text{Half-life} = \ln 2 / k$$

## In vitro drug-drug interaction studies for five major CYPs (fDDI)

*CYP1A2* Test compounds (3 μM) were pre-incubated with recombinant CYP1A2 (Baculosome lot#21198 Invitrogen, 50 pmol P450/ml) in 100 mM $K^+$Phosphate Buffer (pH 7.4) and 10 μM Vivid blue 1A2 probe (Invitrogen) as a substrate for 5 minutes at 30°C. Reaction was initiated by adding a solution of a warmed NADPH-regenerating system A, which consists of 0.50 mM NADP and 10 mM $MgCl_2$, 6.2 mM DL-Isocitric acid and 0.5U/ml Isocitric Dehydrogenase (ICD). Plates were placed in the plate reader at 30 °C and were taken readings every 1.5 minutes, with a 10 second shake in between each reading for 15 cycles. Wavelengths of excitation/emission were 408/465 nm, respectively.

*CYP2C9* Test compounds (3 μM) were pre-incubated with recombinant CYP2C9 (Baculosome lot#20967 Invitrogen, 50 pmol P450/ml) in 100 mM $K^+$Phosphate Buffer (pH 7.4) and 30 μM MFC probe (Gentest) as a substrate for 5 minutes at 37 °C. Reaction was initiated by adding a solution of the warmed NADPH-regenerating system A. Plates were placed in the plate reader at 37°C and were taken readings every 2.0 minutes, with a 10 second shake in between each reading for 15 cycles. Wavelengths of excitation /emission were 408 /535 nm, respectively.

*CYP2C19* Test compounds (3 μM) were pre-incubated with recombinant CYP2C19 (Baculosome lot#20795 Invitrogen, 5 pmol P450/ml) in 100 mM $K^+$Phosphate Buffer (pH 7.4) and 10 μM Vivid blue 2C19 probe (Invitrogen) as a substrate for 5 minutes at 37°C. Reaction was initiated by adding a solution of the warmed NADPH-regenerating system A. Plates were placed in the plate reader at 37°C and were taken readings every 1.5 minutes with a 10 second shake in between each reading for 15 cycles. Wavelengths of excitation /emission were 408 /465 nm, respectively.

*CYP2D6* Test compounds (3 μM) were pre-incubated with recombinant CYP2D6 (Baculosome lot#21248 Invitrogen, 20 pmol P450/ml) in 100 mM $K^+$Phosphate Buffer (pH 7.4) and 1 μM 3-[2-(N,N-diethyl-N-methylammonium)ethyl]-7-methoxy-4-methylcoumarin (AMMC) probe (Gentest) as a substrate for 5 minutes at 37 °C. Reaction was initiated by adding a solution of a warmed NADPH-regenerating system B, which consists of 0.03 mM NADP and 10 mM $MgCl_2$, 6.2 mM DL-Isocitric acid and 0.5 U/ml ICD. Plates were placed in the plate reader at 37°C and were taken readings every 2.0 minutes with a 10 second shake in between each reading for 15 cycles. Wavelengths of excitation /emission were 400 /465 nm, respectively.

*CYP3A4* Test compounds (3 μM) were pre-incubated with recombinant CYP3A4 (Baculosome lot#20814 Invitrogen, 5 pmol P450/ml) in 100 mM $K^+$Phosphate Buffer (pH 7.4) and 2 μM Vivid Red probe (Invitrogen) as a substrate for 5 minutes at 30 °C. Reaction was initiated by adding a solution of the warmed NADPH-regenerating system A. Plates were placed in the plate reader at 30 °C and were taken readings minimum intervals with a 10 second shake in between

each reading for 15 cycles. Wavelengths of excitation /emission were 530 /595 nm, respectively.
Drug-drug interaction was evaluated by the rate of metabolite formation calculated with a slope (Time vs. Fluorescence units) in the linear region or the percentage of inhibition by test compounds calculated by the following equation. Inhibition % = $\{(v_o - v_i)/v_o\}*100$, wherein $v_o$ is a rate of control reaction (no test compounds) and $v_i$ is a rate of reaction in the presence of test compound.

**IHERG assay**

Human ether a-go-go related gene (HERG) transfected HEK293 cells are prepared and cultured in-house. The methodology for stable transfection of this channel in HEK cells can be found elsewhere (Z.Zhou et al., 1998, Biophysical journal, 74, 230-241). On the day of experimentation, the cells are harvested from culture flasks and stored as cell suspension in a standard external solution (see below of its composition), in the room atmosphere of 23 ˚C. Cells are studied between 0.5-5 hours after harvest.

HERG currents are studied using a standard patch clamp technique of the whole-cell mode. During the experiment, the cells are superfused with a standard external solution of the following composition;(mM) NaCl, 130; KCl, 4; $CaCl_2$, 2; $MgCl_2$, 1; Glucose, 10; HEPES, 5; pH 7.4 with NaOH. Whole-cell recordings is made using a patch clamp amplifier and patch pipettes which have a resistance of 1-3MOhm when filled with the standard internal solution of the following composition; (mM); KCl, 130; MgATP, 5; $MgCl_2$, 1; HEPES, 10; EGTA 5, pH 7.2 with . KOH. Only those cells with access resistances below 10 MOhm and seal resistances over 1GOhm are accepted for further experimentation. Series resistance compensation is applied up to a maximum of 80% without any leak subtraction. Following the achievement of whole cell configuration and sufficient time for cell dialysis with pipette solution (>5 min), the membrane is depolarized from a holding potential of - 80 mV to + 30mV for 1000 ms followed by a descending voltage ramp (rate 0.5 mV $msec^{-1}$) back to the holding potential. This depolarization and ramp is applied to the cells continuously every 4 seconds (0.25 Hz). The amplitude of the peak current elicited around -40 mV during the ramp is measured. Once stable evoked current responses of minimal changes in the amplitude are obtained in the external solution, the test compound is applied for 10-20 minutes with multiple dosing in a single cell. The cells are also exposed to high dose of dofetilide (5 μM), a specific IKr blocker, to evaluate the insensitive endogenous current.

All experiments are performed at 23+/-1 ˚C. Evoked membrane currents are recorded online on a computer, filtered at 500-1000 Hz (Bessel -3dB) and sampled at 1-2 KHz. Osmolarity and pH change induced by the test compound in external solution will be examined at the highest concentration.

The arithmetic mean of these ten values of peak current is calculated under control conditions and in the presence of drug. Percent decrease of $I_N$ in each experiment is obtained by the normalized current value using the following formula: $I_N = (I_C - I_D)/(I_C - I_{dof}) \times 100$, where $I_C$ is the mean current value under control conditions, $I_D$ is the mean current value in the presence of test compound and $I_{dof}$ is the mean current value in dofetilide application. Separate experiments are performed and pooled data of arithmetic mean from each experiment is defined as the result of the study.

**Bioavailability in rat**

Adult rats of the Sprague-Dawley strain were used. One to two days prior to the experiments all rats were prepared by cannulation of the right jugular vein under anesthesia. The cannula was exteriorized at the nape of the neck. Blood samples (0.2-0.3 mL) were drawn from the jugular vein at intervals up to 24 hours after intravenous or oral administrations of the test compound. The samples were frozen until analysis. Bioavailability was assessed by calculating the quotient between the area under plasma concentration curve (AUC) following oral administration or intravenous administration.

**Bioavailability in dog**

Adult Beagle dogs were used. Blood samples (0.2-0.5 mL) were drawn from the cephalic vein at intervals up to 24 hours after intravenous or oral administrations of the test compound. The samples were frozen until analysis. Bioavailability was assessed by calculating the quotient between the area under plasma concentration curve (AUC) following oral administration or intravenous administration.

**Plasma protein binding**

Plasma protein binding of the test compound (1 μM) was measured by the method of equilibrium dialysis using 96-well plate type equipment. Spectra-Por®, regenerated cellulose membranes (molecular weight cut-off 12,000-14,000, 22 mm x 120 mm) were soaked for over night in distilled water, then for 20 minutes in 30% ethanol, and finally for 15 minutes in dialysis buffer (Dulbecco's phosphate buffered saline, pH7.4). Frozen plasma of human, Sprague-Dawley

rats, and Beagle dogs were used. The dialysis equipment was assembled and added 150 μL of compound-fortified plasma to one side of each well and 150 μL of dialysis buffer to the other side of each well. After 4 hours incubation at 37 °C for 150 r.p.m, aliquots of plasma and buffer were sampled. The compound in plasma and buffer were extracted with 300 μL of acetonitrile containing internal standard compounds for analysis. The concentration of the compound was determined with LC/MS/MS analysis.

The fraction of the compound unbound was calculated by the following equation:

$$fu = 1-\{ ( [plasma]_{eq} - [buffer]_{eq} ) / ( [plasma]_{eq})\}$$

wherein $[plasma]_{eq}$ and $[buffer]_{eq}$ are the concentrations of the compound in plasma and buffer, respectively.

**Aqueous solubility**

Aqueous solubility in the mediums (a)-(c) was determined by following method:

> Whatman mini-UniPrep chambers (Clifton, NJ, USA) containing more than 0.5 mg of compound and 0.5 mL of each medium were shaken overnight (over 8 hours) at room temperature. All samples were filtered through a 0.45 μm Polyvinylidene Difluoride (PVDF) membrane into the Whatman mini-UniPrep plunger before analysis. The filtrates were assayed by HPLC.

<medium>(a) Simulated gastric fluid with no enzyme (SGN) at pH 1.2: Dissolve 2.0 g of NaCl in 7.0 mL of 10 M HCl and sufficient water to make 1000 mL; (b) Phosphate buffer saline (PBS) at pH 6.5: Dissolve 6.35 g of $KH_2PO_4$, 2.84 g of $Na_2HPO_4$ and 5.50 g of NaCl in sufficient water to make 1000 mL, adjusting the pH to 6.5; (c) 3.94 mg of sodium taurocholate (NaTC) and 1.06 mg of 1-palmitoyl-2-oleyl-L-phosphatidylcholine (POPC) in 1 mL of PBS (pH 6.5).

**Estimation of hepatic clearance using the metabolic stability in human hepatocytes**

Tested compounds (1 μM) were incubated statically with hepatocytes from human at 37 °C in a 95 % air/ 5 % $CO_2$ with target cell density of $0.5 \times 10^6$ cells/ml and a total volume of 50 μL. Incubation was stopped at each time point by the addition of ice-cold acetonitrile (ACN). Aliquots of samples were mixed with 10 % ACN containing an internal standard for LC/MS/MS analysis. After samples were sonicated for 10 minutes, samples were centrifuged at 2,000 rpm for 15 minutes, and then the supernatant was transferred to the other plates for analysis. The compound concentrations in supernatant were measured by LC/MS/MS system.

The disappearance rates of tested compounds were obtained by plotting the common logarithm of the peak area ratio of compounds / internal standard versus time. The slope of the line of best fit through the points yielded the rate of metabolism ($k_e$). This value was scaled to take hepatocellularity, liver and body weight into account to give an intrinsic clearance value ($CL_{int}$) in ml/min/kg as illustrated in Equation 1. Hepatic clearance ($CL_h$) was predicted from this intrinsic clearance value using the parallel tube model as shown in Equation 2. The predicted clearance divided by the hepatic blood flow ($Q_h$) afforded the extraction ratio ($E_h$) (Equation 3).

Equation 1: $k_e$ x (g liver/kg body weight)x(ml incubation/ number of cells in incubation)x(cells/g liver)

Equation 2: $$CL_h = Q_h \times \{ 1 - \exp (-CL_{int} / Q_h) \}$$

Equation 3: $$E_h = CL_h / Q_h$$

Wherein, "gliver weight /kg body weight" is 21, "Cells / g liver" is $1.2 \times 10^8$, "ml incubation/ number of cells in incubation" is $2.0 \times 10^{-6}$, and $Q_h$ is 20 ml/min/kg.

Supposing that hepatic metabolism is the main route of drug elimination, systemic exposure ($AUC_{po}$) after oral administration is calculated using Equation 4.

$$\text{Equation 4} \qquad AUC_{po} = Dose \times (1-E_h) / CL_h$$

**Method for assaying the compounds phototoxic potential:**

The phototoxic potential was measured in the strict accordance with method described in the OECD Guidelines for the Testing of Chemicals 432 (2002). Chlorpromazine (CPZ) and Sodium n-Dodecyl Sulfate (SDS) were used as positive and negative controls, respectively.

Balb/3T3, clone 31 cells (ATCC, CCL-163) were seeded into 96-wells plates (Nunc, 167008) at a density of 1×104 cells/well. Cells were incubated under a standard condition (37°C. a humidified atmosphere of 95% air and 5% CO2) within the culture medium-DMEM (GIBCO; cat#11885-084) for 24 hour. Following the incubation, the culture medium-DMEM was discarded and the cells were washed carefully with 150 μl of Earle's Balanced Salt Solution (EBSS; Sigma, Cat#E3024), then added 100 μl solution of the test compound in EBSS or solvent control (EBSS contained 1 % dimethylsulphoxide or 1% ethanol). The plate was prepared in duplicate. All the plates were incubated under the standard condition for 60 min in the dark. One of the duplicated plates was used for determination of cytotoxicity (-Irr) and kept at room temperature in the dark for 50 min. For the determination of photocytotoxicity (+Irr), another one was exposed to the sun simulator (UVA irradiance: 1.7mW/cm2; SOL500, Dr. Honle UV Technology, Germany) for 50 min (UVA dose = 5 joules / cm2). Then the solutions were discarded from the two plates and immediately washed with 150 μl of EBSS with care. The cells were further incubated with 150 μl/well of DMED medium for 18 - 22 hr.

After the incubation, the culture medium was discarded, the cells were washed carefully with 150 μl of EBSS and then immediately incubated with 100 μl/well of a 50 μg/ml of neutral red (NR) (3-amino-7-dimethylamino-2-methylphenazine hydrochloride, Kanto Chemical Co., Inc., Japan) in DMEM without serum for 3 hour under the standard condition. After incorporation of neutral red into the cell lysosomes, the NR-DMED medium was discarded and the cells were washed carefully with 150 μl of EBSS. The exact 150 μl of ethanol/acetic acid/water (50:1:49) was added to each well of plate and the extraction was performed for 10 minutes by gently shaking at room temperature. Then optical density (OD) of the NR extract was measured at 540 nm using a spectrophotometer (Plate-reader, POLARstar OPTIMA; BMG Labtechnologies, Germany). The OD values were used to calculate the mean photo effect (MPE) value using OECD provided software "3T3 NRU Phototox". (Version 2.0, Federal Institute for Risk Assessment, Germany). The results for the control (CPZ and SDS) were used for the quality assurance of the assay.

MPE value < 0.1 was evaluated as "no-phototoxicity"; MPE value ≥ 0.1 and < 0.15 was evaluated as "probable phototoxicity" and MPE value ≥ 0.15 was evaluated as "phototoxicity".

**Examples**

The following examples are provided for the purpose of further illustration only and are not intended to be limitations on the disclosed invention. Unless stated on otherwise in the following examples, general experimental conditions are as follows: all operations were carried out at room or ambient temperature, that is, in the range of 18-25 °C; evaporation of solvent was carried out using a rotary evaporator under reduced pressure with a bath temperature of up to 60 °C; reactions were monitored by thin layer chromatography (TLC) and reaction times are given for illustration only; melting points (mp) given are uncorrected (polymorphism may result in different melting points); the structure and purity of all isolated compounds were assured by at least one of the following techniques: TLC (Merck silica gel 60 $F_{254}$ precoated TLC plates or Merck $NH_2$ gel (an amine coated silica gel) $F_{254s}$, precoated TLC plates), mass spectrometry, nuclear magnetic resonance spectra (NMR), infrared absorption spectra (IR) or microanalysis. Yields are given for illustrative purposes only. Flash column chromatography was carried out using Biotage KP-SIL (40-63 μm), Biotage KP-NH (an amine coated silica gel) (40-75 μM) or Wako silica gel 300HG (40-60 μM). Preparative TLC was carried out using Merck silica gel 60 $F_{254}$ precoated TLC plates (0.5 or 1.0 mm thickness). All Mass data was obtained in Low-resolution mass spectral data (ESI) using ZMD™ or ZQ™ (Waters) and mass spectrometer. NMR data were determined at 270 MHz (JEOL JNM-LA 270 spectrometer) or 300 MHz (JEOL JNM-LA300 spectrometer) using deuterated chloroform (99.8%) or dimethylsulfoxide (99.9%) as solvent unless indicated otherwise, relative to tetramethylsilane (TMS) as internal standard in parts per million (ppm); conventional abbreviations used are: s = singlet, d = doublet, m = multiplet, dd = doublet of doublet, br.s = broad singlet, etc. IR spectra were measured by a Fourier transform infrared spectrophotometer (Shimazu FTIR-8300). Optical rotations were measured using a JASCO DOP-370 and P-1020 Digital Polarimeter (Japan Spectroscopic CO, Ltd.).

**Example 1**

**<u>8-(3,4-Dihydro-2*H*-chromen-4-ylamino)-*N*,*N*,2,3-tetramethylimidazo[1,2-*a*]pyridine-6-carboxamide</u> (example 1-1)**

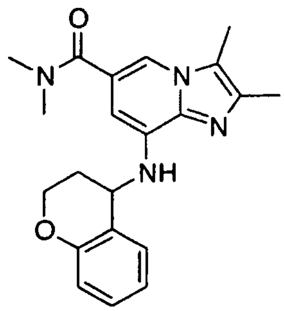

<u>STEP 1: Isopropyl 8-(3,4-dihydro-2*H*-chromen-4-ylamino)-2,3-dimethylimidazo[1,2-*a*]pyridine-6-carboxyl ate</u>

To a suspension of isopropyl 8-amino-2,3-dimethylimidazo[1,2-*a*] pyridine-6-carboxylate (8.00 g, 32.3 mmol, WO02/20523), sodium iodide (2.42 g, 16.2 mmol) and potassium carbonate (15.6 g, 113 mmol) in acetone (100 mL) was added a solution of 4-chlorochromane (10.9 g, 64.6 mmol, WO00/78751) in acetone (20 mL) at room temperature and the reaction mixture was stirred at reflux temperature for 24 hours. The reaction mixture was quenched with water and extracted with dichloromethane (80 mL x 2). The combined extracts were washed with brine, dried over magnesium sulfate, and concentrated in vacuum. The residue was purified by column chromatography on silica gel (n-hexane : ethyl acetate = 7 : 1 as eluent) to afford the titled compound as a white solid (6.37 g, 52%).
$^1$H NMR (300 MHz, $CDCl_3$) δ: 8.06-8.03 (m, 1H), 7.34-7.28 (m, 1 H), 7.24-7.17 (m, 1H), 6.93-6.83 (m, 2H), 6.79 (s, 1 H), 5.44 (d, J = 6.61 Hz, 1 H), 5.38-5.23 (m, 1 H), 4.89-4.79 (m, 1H). 4.34-4.25 (m, 2H), 2.43 (s, 3H), 2.37 (s, 3H), 2.29-2.20 (m, 2H), 1.41 (d, J = 5.87 Hz, 6H) ppm.
MS: 380 $(M+H)^+$.

<u>STEP 2: 8-(3,4-Dihydro-2*H*-chromen-4-ylamino)-2,3-dimethylimidazo[1,2-*a*]pyridine-6-carboxylic acid h ydrochloride</u>

A mixture of isopropyl 8-(3,4-dihydro-2*H*-chromen-4-ylamino)-2,3-dimethyl imidazo[1,2-*a*]pyridine-6-carboxylate (2.00 g, 5.27 mmol, Step 1), methanol (100 mL), and 2 M sodium hydroxide solution (14.2 mL) was stirred at 60 ˚C for 5 hours. The reaction mixture was cooled, and water (50 mL) was added to the mixture. The pH was adjusted to pH = 3 by addition of 2 M hydrochloric acid. The mixture was extracted with dichloromethane : methanol =10:1 (50 mL x 2) and ethyl acetate (30 mL). The combined organic layer was washed with brine, dried over sodium sulfate, and evaporated in vacuum to afford the titled compound as a white solid (1.70 g, 86%).
$^1$H NMR (300 MHz, DMSO-$d_6$) δ: 8.33 (s. 1H). 7.28-7.20 (m, 3H), 7.05-6.94 (m, 1H), 6.92-6.84 (m, 2H), 5.10-4.99 (m, 1H), 4.36-4.20 (m, 2H), 2.51 (s, 3H), 2.41 (s, 3H), 2.17-2.00 (m, 2H) ppm. ($-CO_2H$ and HCl salt were not observed)
MS: 338 $(M + H)^+$, 336 $(M - H)^-$.

<u>STEP 3: 8-(3,4-Dihydro-2*H*-chromen-4-ylamino)-*N*,*N*,2,3-tetramethylimidazo[1,2-*a*]pyridine-6-carboxami de</u>

To a stirred mixture of 8-(3,4-dihydro-2H-chromen-4-ylamino)-2,3-dimethyl imidazo[1,2-*a*]pyridine-6-carboxylic acid hydrochloride (1.00 g, 2.68 mmol, Step 2) and dimethylamine hydrochloride (362 mg, 4.44 mmol) in dichloromethane (50 mL) were added 1-(3-dimethylaminopropyl)-3-ethylcarbodiimide hydrochloride (EDCI) (852 mg, 4.44 mmol), 1-hydroxybenzotriazole hydrate (HOBt) (680 mg, 4.44 mmol), and triethylamine (1.64 mL) at 0 ˚C. After being stirred at room temperature overnight, the reaction mixture was quenched with water (50 mL). The mixture was extracted with dichloromethane (50 mL x 2) and the combined extracts were washed with brine, dried over sodium sulfate, and evaporated in vacuum. The residue was purified by column chromatography on silica gel eluting with ethyl acetate to afford the titled compound as a white solid (992 mg, 92%).
$^1$H NMR (300 MHz, $CDCl_3$) δ: 7.44 (d, *J* = 1.32 Hz, 1H), 7.34-7.14 (m, 2H), 6.91-6.84 (m, 2H), 6.21 (s, 1H), 5.48 (d, *J* = 6.59 Hz, 1H). 4.78-4.72 (m, 1 H), 4.34-4.22 (m, 2H), 3.11 (s, 6H), 2.36 (s, 6H), 2.32-2.11 (m, 2H) ppm.
MS: 365 $(M + H)^+$.

The fraction-1 (400 mg) and fraction-2 (418 mg) were prepared from racemic 8-(3,4-dihydro-2*H*-chromen-4-ylamino)-*N*,*N*,2,3-tetramethylimidazo[1,2-*a*]pyridine-6-carboxamide (992 mg) by HPLC as follows.

Resolution condition

Column: CHIRALPAK® AD-H (20 mm I.D. x 250 mm, DAICEL)
Mobile phase: n-Hexane / Ethanol / Diethylamine (80 / 20 / 0.1)
Flow rate: 18.9 mL/min

(-)-8-(3.4-Dihvdro-2*H*-chromen-4-ylamino)-*N*,*N*,2,3-tetramethylimidazo[1,2-*a*]pyridine-6-carboxamide (fraction-1) (example 1-2)

NMR: spectrum data were identical with those of the racemate
Optical rotation: $[\alpha]_D^{24}$ = -13.0 ° (c = 1.00, Methanol)
Retention time: 8 min

(+)-8-(3,4-Dihydro-2*H*-chromen-4-ylamino)-*N*,*N*,2,3-tetramethylimidazo[1,2-*a*]pyridine-6-carboxamide (fraction-2) (example 1-3)

NMR: spectrum data were identical with those of the racemate
Optical rotation: $[\alpha]_D^{23}$ = +13.4° (c = 1.00, Methanol)
Retention time: 11 min

**Example2**

**8-(3,4-Dihydro-2*H*-chromen-4-ylamino)-*N*-(2-hydroxyethyl)-*N*,2,3-trimethylimidazo[1,2-*a*]pyridine-6-carboxamide (example 2-1)**

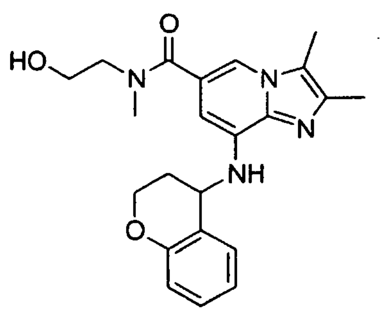

The title compound was prepared in 86% yield (1.11 g) from 8-(3,4-dihydro-2*H*-chromen-4-ylamino)-2,3-dimethylimidazo[1,2-*a*]pyridine-6-carboxylic acid hydrochloride (1.10 g, 2.94 mmol) and 2-(methylamino)ethanol (367 mg, 4.89 mmol) by the same manner as the preparation of 8-(3,4-dihydro-2*H*-chromen-4-ylamino)-*N*,*N*,2,3-tetramethylimidazo [1,2-*a*]pyridine-6-carboxamide (Step 3 of Example 1).
$^1$H NMR (300 MHz, $CDCl_3$) δ: 7.52 (s, 1H), 7.31-7.17 (m, 2H), 6.91-6.84 (m, 2H), 6.25 (s, 1H), 5.51 (d, *J* = 6.97, 1 H), 4.79-4.73 (m, 1H), 4.30-4.26 (m, 2H), 4.01-3.82 (m, 2H), 3.79-3.60 (m, 2H), 3.14 (s, 3H), 2.36 (s, 6H), 2.29-2.17 (m, 2H) ppm. (-OH was not observed)
MS: 395 $(M+H)^+$.

The fraction-1 (511 mg) and fraction-2 (532 mg) were prepared from racemic 8-(3,4-dihydro-2*H*-chromen-4-ylamino)-*N*-(2-hydroxyethyl)-*N*,2,3-trimethylimidazo[1,2-*a*]pyridine-6-carbox amide (1.11 g) by HPLC as follows.

Resolution condition

Column: CHIRALPAK®AD-H (20 mm I.D. x 250 mm, DAICEL)
Mobile phase: n-Hexane / Ethanol / Diethylamine (85/15/0.1)
Flow rate: 18.9 mUmin

(-)-8-(3,4-Dihydro-2*H*-chromen-4-ylamino)-*N*-12-hydroxyethyl)-*N,*2,3-trimethylimidazo[1,2-*a*]pyridine-6-car boxamide (fraction-1) (example 2-2)

NMR: spectrum data were identical with those of the racemate
Optical rotation: $[\alpha]_D^{23}$ = -13.6 ° (c = 1.00, Methanol)
Retention time: 10 min

(+)-8-(3,4-Dihydro-2*H*-chromen-4-ylamino)-*N*-(2-hydroxyethyl)-*N*-2,3-trimethylimidazo[1,2-*a*]pyridine-6-car boxamide (fraction-2) (example 2-3)

NMR: spectrum data were identical with those of the racemate
Optical rotation: $[\alpha]_D^{23}$ = +15.0° (c = 1.00, Methanol)
Retention time: 13 min

**Example 3**

**8-(3,4-Dihydro-1*H*-isochromen-4-ylamino)-*N*,*N*,2,3-tetramethylimidazol[1,2-*a*]pyridine-6-carboxamid e (example 3-1)**

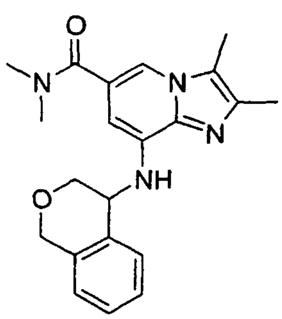

STEP 1: 4-Chloro-3,4-dihydro-1*H*-isochromene

A solution of thionyl chloride (14.3 mL, 196 mmol) in diethyl ether (20 mL) was added to a mixture of 3,4-dihydro-1*H*-isochromen-4-ol (5.89 g, 39.2 mmol, WO04/024081) and pyridine (1.0 mL) in diethyl ether (100 mL). The reaction mixture was stirred at room temperature overnight. After the mixture was evaporated in vacuum, the residue was poured into ice and the mixture was extracted with diethyl ether (50 mL x 2). The combined extracts were washed with brine, dried over magnesium sulfate, and concentrated in vacuum to afford the titled compound as yellow oil (6.55 g, 99%).
$^1$H NMR (270 MHz, $CDCl_3$) δ 7.60-7.40 (m, 1H). 7.40-7.20 (m, 2H), 7.10-6.90 (m, 1H), 5.18-5.08 (m, 1H), 4.95-4.72 (m, 2H), 4.35-4.10 (m, 2H) ppm.

STEP 2: Isopropyl 8-(3,4-dihydro-1*H*-isochromen-4-ylamino)-2,3-dimethylimidazo[1,2-*a*]pyridine-6-carbo xylate

To a suspension of isopropyl 8-amino-2,3-dimethylimidazo[1,2-*a*] pyridine-6-carboxylate (6.40 g, 25.9 mmol, WO02/20523), sodium iodide (1.95 g, 13.0 mol) and potassium carbonate (7.16 g, 51.8 mmol) in 2-propanol (50 mL) was added dropwise a solution of 4-chloro-3,4-dihydro-1*H*-isochromene (4.37 g, 25.9 mmol, Step 1) in 2-propanol (3.0 mL) at 70˚C and the reaction mixture was stirred at 70 ˚C overnight. To the mixture, a solution of 4-chloro-3,4-dihydro-1*H*-isochromene (2.19 g, 13.0 mmol) in 2-propanol (1.0 mL) was added and the reaction mixture was stirred at 70˚C for 24 hours. After cooled to room temperature, the mixture was evaporated in vacuum. The residue was treated with water and extracted with dichloromethane (50 mL x 2). The combined extracts were washed with brine, dried over magnesium sulfate, and concentrated in vacuum. The residue was purified by column chromatography on silica gel (*n*-hexane : ethyl acetate = 7 : 1 to 3 : 1 as eluent) to afford the titled compound as a yellow solid (2.04 g, 21%).
$^1$H NMR (270 MHz, $CDCl_3$) δ: 8.04 (s, 1H), 7.49-7.41 (m, 1H), 7.32-7.16 (m, 2H), 7.07-7.00 (m, 1H), 6.80 (s, 1 H), 5.47 (d, *J* = 9.40 Hz, 1H), 5.36-5.21 (m, 1 H), 4.96-4.70 (m, 3H), 4.17-3.97 (m, 2H), 2.42 (s, 3H), 2.36 (s, 3H), 1.40 (d, *J* = 6.61 Hz, 6H) ppm.
MS: 380 $(M+H)^+$.

STEP 3: 8-(3,4-Dihvdro-1*H*-isochromen-4-ylamino)-2,3-dimethylimidazo[1,2-*a*]pyridine-6-carboxylic acid hydrochloride

The title compound was prepared in quantitative yield (2.05 g) from isopropyl 8-(3,4-dihydro-1*H*-isochromen-4-ylamino)-2,3-dimethylimidazo[1,2-*a*]pyridine-6-carboxylate (2.04 g, 5.38 mmol, Step 2) by the same manner as the preparation of 8-(3,4-dihydro-2*H*-chromen-4-ylamino)-2,3-dimethylimidazo[1,2-*a*]pyridine-6-carboxylic acid hydrochloride (Step 2 of Example 1).
$^1$H NMR (300 MHz, DMSO-$d_6$)δ: 8.19 (s, 1H), 7.42-7.08 (m, 4H), 6.97 (br.s, 1H), 6.15-5.89 (m, 1H), 5.04-4.89 (m, 1H), 4.89-4.57 (m, 2H), 4.06-3.89 (m, 2H), 2.43 (s, 3H), 2.31 (s, 3H) ppm. (-$CO_2$H and HCl salt were not observed)

STEP 4: 8-(3,4-Dihydro-1*H*-isochromen-4-ylamino)-*N*,*N*,2,3-tetramethylimidazo[1,2-*a*]pyridine-6-carboxa mide

The title compound was prepared in 59% yield (46 mg) from 8-(3,4-dihydro-1*H*-isochromen-4-ylamino)-2,3-dimethylimidazo[1,2-*a*]pyridine-6-carboxylic acid hydrochloride (80 mg, 0.22 mmol, Step 3) and dimethylamine hydrochloride (65 mg, 0.36 mmol) by the same manner as the preparation of 8-(3,4-dihydro-2*H*-chromen-4-ylamino)-*N*,*N*,2,3-tetramethylimidazo[1,2-*a*]pyridine-6-carboxamide (Step 3 of Example 1).
$^1$H NMR (270 MHz, DMSO-$d_6$) δ: 7.61 (s, 1H), 7.43-7.03 (m, 4H), 6.40 (s, 1H), 5.69 (d, *J* = 9.99 Hz, 1H), 5.07-4.88 (m, 1H), 4.88-4.61 (m, 2H), 4.09-3.83 (m, 2H), 2.98 (s, 6H), 2.34 (s, 3H), 2.24 (s, 3H) ppm.
MS: 365 $(M+H)^+$.

The fraction-1 (6.4 mg) and fraction-2 (9.3 mag) were prepared from racemic 8-(3,4-dihydro-1*H*-isochromen-4-ylamino)-*N*,*N*,2,3-tetramethylimidazo[1,2-*a*] pyridine-6-carboxamide (21 mg) by HPLC as follows.

Resolution condition

Column: CHIRALPAK® AD-H (20 mm I.D. x 250 mm, DAICEL)
Mobile phase: n-Hexane / Ethanol / Diethylamine (85/15/0.1)
Flow rate: 18.9 mL/min

(-)-8-(3,4-Dihydro-1*H*-isochromen-4-ylamino)-*N*,*N*,2,3-tetramethylimidazo[1,2-*a*]pyridine-6-carboxamide (fraction-1) (example 3-2)

NMR: spectrum data were identical with those of the racemate
Optical rotation: $[\alpha]_D^{24}$ = -19.4 ˚(c = 1.00, Methanol)
Retention time: 14 min

(+)-8-(3,4-Dihydro-1*H*-isochromen-4-ylamino)-*N*,*N*,2,3-tetramethylimidazo[1,2-*a*]pyridine-6-carboxamide (fraction-2) (example 3-3)

NMR: spectrum data were identical with those of the racemate
Optical rotation: $[\alpha]_D^{24}$ = +20.4 ° (c = 1.00, Methanol)
Retention time: 19 min

**Example 4**

**8-(3,4-Dihydro-1*H*-isochromen-4-ylamino)-*N*-(2-hydroxyethyl)-*N*,2,3-trimethylimidazo[[1,2-*a*]pyridine -6-carboxamide (example 4-1)**

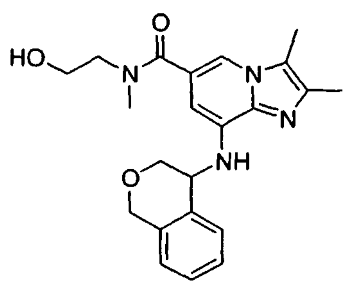

A mixture of isopropyl 8-(3,4-dihydro-1 H-isochromen-4-ylamino)-2,3-dimethylimidazo[1,2-*a*]pyridine-6-carboxylate (270 mg, 0.71 mmol, Step 2 of Example 3), 2-(methylamino)ethanol (530 mg, 7.1 mmol) and sodium cyanide (2.0 mg, 0.04 mmol) was refluxed in tetrahydrofran (2.0 mL) for 14 hours. After cooled to room temperature, the solvent was evaporated in vacuum. The residue was purified by column chromatography on NH gel (dichloromethane to dichloromethane : methanol = 40 : 1 as eluent) to afford the titled compound as a white solid (100 mg, 37%).
$^1$H NMR (300 MHz, $CDCl_3$) δ: 7.55-7.49 (m, 1H), 7.44 (d, *J* = 6.60 Hz, 1H), 7.34-7.16 (m, 3H), 7.04 (d, *J* = 6.60 Hz, 1H), 6.28 (br.s, 1H), 5.65-5.42 (m, 1H), 4.94-4.69 (m, 3H), 4.10-4.01 (m, 2H), 3.97-3.82 (m, 2H), 3.80-3.61 (m, 2H), 3.14 (s, 3H), 2.36 (s, 6H) ppm.
MS: 395 $(M+H)^+$.

The fraction-1 (22 mg) and fraction-2 (20 mg) were prepared from racemic 8-(3,4-dihydro-1*H*-isochromen-4-ylamino)-*N*-(2-hydroxyethy))-*N*,2,3-trimethytimidazo[1,2-*a*]pyridine-6-carb oxamide (63 mg) by HPLC as follows.

Resolution condition

Column: CHIRALPAK® AD-H (20 mm I.D. x 250 mm, DAICEL)
Mobile phase: n-Hexane / Ethanol / Diethylamine (80 / 20 / 0.1)
Flow rate: 18.9 mL/min

(-)-8-(3,4-Dihydro-1*H*-isochromen-4-ylamino)-*N*-(2-hydroxyethyl)-*N*,2,3-trimethylimidazo[1,2-*a*]pyridine-6-c arboxamide (fraction-1) (example 4-2)

NMR: spectrum data were identical with those of the racemate
Optical rotation: $[\alpha]_D^{23}$= -39.8 ° (c = 1.00, Methanol)
Retention time: 11 min

(+)-8-(3,4-Dihydro-1*H*-isochromen-4-ylamino)-*N*-(2-hydroxyethyl)-*N*,2,3-trimethylimidazo[1,2-*a*]pyridine-6-carboxamide (fraction-2) (example 4-3)

NMR: spectrum data were identical with those of the racemate
Optical rotation: $[\alpha]_D^{24}$ = +40.8 ° (c = 1.00, Methanol)
Retention time: 12 min

**Example 5**

***N*-(2-Hydroxyethyl)-*N*,2,3-trimethyl-8-[(5-methyl-3,4-dihydro-2*H*-chromen-4-yl)amino]imidazo[1,2-*a*] pyridine-6-carboxamide (example 5-1)**

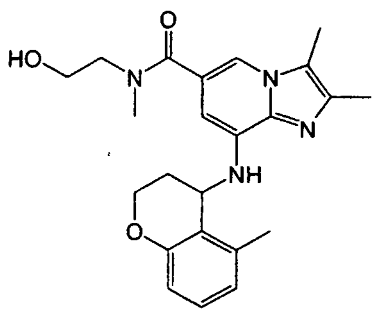

STEP 1: 4-Chloro-5-methylchromane

A solution of thionyl chloride (6.0 mL, 83 mmol) in diethyl ether (6 mL) was added to a mixture of 5-methylchroman-4-ol (2.7 g, 17 mmol, Tetrahedron Asym., 1997, 8, 3059.) and pyridine (0.4 mL) in diethyl ether (30 mL). The reaction mixture was stirred at room temperature for 8 hours. After the mixture was evaporated in vacuum, the residue was poured into ice and the mixture was extracted with ethyl acetate (50 mL x 2). The combined extracts were washed with brine, dried over magnesium sulfate, and concentrated in vacuum to afford the titled compound as yellow oil (3.2 g, quantitative yield).
$^1$H NMR (300 MHz, $CDCl_3$) δ: 7.21-7.04 (m, 1H), 6.86-6.62 (m, 2H), 5.36-5.17 (m, 1H). 4.59-4.43 (m, 1 H), 4.43-4.30 (m, 1H), 2.41 (s, 3H), 2.57-2.24 (m, 2H) ppm.

STEP 2: Methyl 2,3-dimethyl-8-[(5-methyl-3.4-dihydro-2*H*-chromen-4-yl)amino]imidazo[1,2-*a*]pyridine-6-carboxylate

A mixture of 4-chloro-5-methylchromane (3.2 g, 16 mmol, Step 1), methyl 8-amino-2,3-dimethylimidazo[1,2-*a*] pyridine-6-carboxylate (2.4 g, 11 mmol, WO02/020523), sodium iodide (0.83 g, 5.5 mol) and potassium carbonate (5.3 g, 38 mmol) in acetone (100 mL) was stirred at reflux for 2 days. After cooled to room temperature, the mixture was quenched with water (50 mL) and extracted with dichloromethane (100 mL x 2). The combined extracts were washed with brine, dried over magnesium sulfate, and concentrated in vacuum. The residue was purified by column chromatography on silica gel (n-hexane : ethyl acetate = 5 : 1 as eluent) to afford the titled compound as a white solid (3.0 g, 74%).
$^1$H NMR (300 MHz, $CDCl_3$) δ: 8.09 (s, 1H), 7.18-7.06 (m, 1H), 6.83-6.68 (m, 3H), 5.36-5.25 (m, 1H), 4.79-4.68 (m, 1H), 4.32-4.17 (m, 2H), 3.96 (s, 3H), 2.41 (s, 3H), 2.36 (s, 3H), 2.22 (s, 3H), 2.48-1.98 (m, 2H) ppm.
MS: 366 $(M+H)^+$.

STEP 3: 2,3-Dimethyl-8-[(5-methyl-3,4-dihydro-2*H*-chromen-4-yl)amino]imidazo[1,2,*a*]pyridine-6-carboxy lic acid

To a mixture of methyl 2,3-dimethyl-8-[(5-methyl-3,4-dihydro-2*H*-chromen-4-yl)amino]imidazo[1,2-*a*]pyridine-6-carboxylate (3.0 g, 8.2 mmol, Step 2) in methanol (30 mL)-tetrahydrofuran (15 mL) was added 2 M sodium hydroxide solution (12 mL) and the reaction mixture was stirred at 50°C for 2 hours. After cooled to room temperature, the mixture was evaporated in vacuum. The residue was treated with water (10 ml) and the pH was adjusted to pH = 6 by addition of 2M hydrochloric acid. The

precipitate was collected by filtration and washed with water (5 mL), acetone (5 mL), and dried to afford the titled compound as a white solid (3.6 g, quantitative yield).
$^1$H NMR (300 MHz, DMSO-$d_6$) δ: 8.08 (s, 1H), 7.21-7.03 (m, 1H), 6.83-6.64 (m, 3H), 5.56 (d, *J* = 7.34 Hz, 1H), 4.86-4.73 (m, 1H), 4.31-4.01 (m, 2H), 2.39 (s, 3H), 2.25 (s, 3H), 2.14 (s, 3H), 2.45-1.86 (m, 2H) ppm.
(-$CO_2$H was not observed)
MS: 352 $(M+H)^+$.

STEP 4: *N*-(2-Hydroxyethyl)-*N*,2,3-triméthyl-8-[(5-methyl-3,4-dihydro-2*H*-chromen-4-yl)amino]imidazo[1, 2-alpyridine-6-carboxamide

To a stirred mixture of 2,3-dimethyl-8-[(5-methyl-3,4-dihydro-2*H*-chromen-4-yl)amino]imidazo[1,2-*a*]pyridine-6-carboxylic acid (2.7 g, 7.7 mmol, Step 3) and 2-(methylamino)ethanol (1.1 g, 15 mmol) in dichloromethane (16 mL) were added 1-(3-dimethylaminopropyl)-3-ethylcarbodiimide hydrochloride (EDCI) (2.9 g, 15 mmol) and 1-hydroxybenzotriazole hydrate (HOBt) (2.1 g, 15 mmol) at 0 °C and the reaction mixture was stirred at room temperature overnight. The reaction mixture was quenched with saturated sodium hydrogencarbonate (30 mL). The mixture was extracted with dichloromethane (50 mL x 2) and the combined extracts were washed with brine, dried over sodium sulfate, and evaporated in vacuum. The residue was purified by column chromatography on silica gel (dichloromethane : methanol = 30 : 1 as eluent) to afford the titled compound as a white solid (2.0 g, 63%).
$^1$H NMR (270 MHz, $CDCl_3$) δ: 7.52 (s, 1H), 7.17-7.06 (m, 1 H), 6.82-6.65 (m, 2H), 6.30 (s, 1H), 5.38 (d, *J* = 5.93 Hz, 1H), 4.73-4.59 (m, 1H), 4.34-4.15 (m, 2H), 4.03-3.84 (m, 2H), 3.84-3.62 (m, 2H), 3.18 (s, 3H), 2.36 (s, 3H), 2.35 (s, 3H), 2.22 (s, 3H), 2.30-1.96 (m, 2H) ppm. (-OH was not observed)
MS: 409 $(M+H)^+$.
IR (KBr)$\nu_{max}$: 3443, 1616, 1557, 1407, 1254, 1095, 1056, 783, 748 $cm^{-1}$.

The fraction-1 (72 mg) and fraction-2 (70 mg) were prepared from racemic *N*-(2-hydroxyethyl)-*N*,2,3-trimethyl-8-[(5-methyl-3,4-dihydro-2*H*-chrome-4-yl)amino]imidazo[1,2-*a*]pyridin e-6-carboxamide (180 mg) by HPLC as follows.

Resolution condition

Column: CHIRALPAK® OD-H (20 mm I.D. x 250 mm, DAICEL)
Mobile phase: *n*-Hexane / Ethanol / Diethylamine (90/10/0.1)
Flow rate: 18.9 mL/min

(-)-*N*-(2-Hydroxyethyl)-*N*,2,3-trimethyl-8-[(5-metyl-3,4-dihydro-2*H*-chromen-4-yl)amino]imidazo[1,2-*a*]pyri dine-6-carboxamide (fraction-1) (example 5-2)

NMR: spectrum data were identical with those of the racemate
Optical rotation: $[a]_D^{24}$ = -6.2° (c = 1.00, Methanol)
Retention time: 11 min

The absolute configuration of the compound of example 5-2 was determined as (*S*)-form by single crystal X-Ray analysis.

(+)-*N*-(2-Hydroxyethyl)-*N*,2,3-trimethyl-8-[(5-methyl-3,4-dihydro-2*H*-chromen-4-yl)amino)imidazo[1,2-*a*]pyr idine-6-carboxamide (fraction-2) (example 5-3)

NMR: spectrum data were identical with those of the racemate
Optical rotation: $[\alpha]_D^{24}$ = +5.8° (c = 1.00, Methanol)
Retention time: 13 min

The absolute configuration of the compound of example 5-3 was determined as (*R*)-form by single crystal X-Ray analysis.

Following **Examples 6 to 19** were prepared according to the procedure described in Step 1-3 of Example 1. $^1$H-NMR was measured with using $CDCl_3$.

| | |
|---|---|
| **Example 6** | **8-[(7-Fluoro-3,4-dihydro-2*H*-chromen-4-yl)amino]-*N*,*N*,2,3.tetramethylimidazo[1,2-*a*]pyridine-6-carboxamide (example 6-1)** |
| 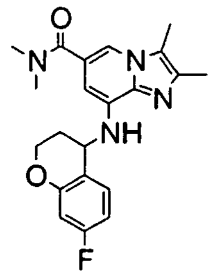 | white solid<br>$^1$H-NMR (300 MHz) δ: 7.45 (s, 1H), 7.36-7.16 (m, 1H), 6.69-6.52 (m, 2H), 6.22 (s, 1H), 5.43 (d, J = 6.6 Hz, 1 H), 4.80-4.67 (m, 1 H), 4.37-4.20 (m, 2H), 3.12 (s, 6H), 2.37 (s, 6H), 2.33-2.05 (m, 2H) ppm.<br>MS m/z: 383 $(M + H)^+$.<br>**Resolution condition**<br>Column: CHIRALPAK® AD-H (20 mm I.D. x 250 mm, DAICEL)<br>Mobile phase: n-Hexane / Ethanol / Diethylamine ( 80 / 20 / 0.1 )<br>Flow rate: 18.9 mL/min<br>**(-)-isomer (example 6-2):** optical rotation: $[\alpha]_D^{24}$ = -12.3 ° (c = 1.00, Methanol), retention time: 8 min<br>**(+)-isomer (example 6-3):** optical rotation: $[\alpha]_D^{25}$ = +10.6 ° (c = 1.00, Methanol), retention time: 12 min |
| **Example 7** | **8-[(7-Fluoro-3,4-dihydro-2*H*-chromen-4-yl)amino]-*N*-(2-hydroxyethyl)-*N*,2, 3-trimethylimidazo[1,2-*a*]pyridine-6-carboxamide (example 7-1)** |
| 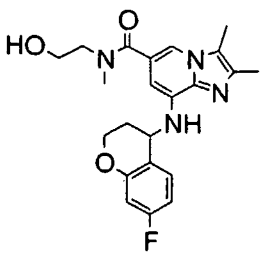 | white solid<br>$^1$H-NMR (270 MHz, $CDCl_3$) δ: 7.52 (s, 1H), 7.38-7.14 (m, 1 H), 6.70-6.49 (m, 2H), 6.25 (s, 1H), 5.45 (d, *J* = 6.6 Hz, 1H), 4.80-4.65 (m, 1H), 4.38-4.19 (m, 2H), 4.01-3.82 (m, 2H), 3.82-3.61 (m, 2H), 3.15 (s, 3H), 2.37 (s, 6H), 2.45-2.01 (m, 2H) ppm. (OH was not observed)<br>MS m/z: 411 $(M - H)^-$.<br>**Resolution condition**<br>Column: CHIRALPAK® AS-H (20 mm I.D. x 250 mm, DAICEL)<br>Mobile phase: *n*-Hexane / Ethanol / Diethylamine (90/10/0.1)<br>Flow rate: 18.9 mL/min<br>**(-)-isomer (example 7-2):** optical rotation: $[c]_D^{24}$ = -10.5 ° (c = 1.00, Methanol), retention time: 8 min<br>**(+)-isomer (example 7-3):** optical rotation: $[\alpha]_D^{24}$ = +12.2 ° (c = 1.00, Methanol), retention time: 10 min |
| **Example 8** | **8-[(5,7-Difluoro-3,4-dihydro-2*H*-chromen-4-yl)amino]-*N*,*N*,2,3-tetramethylimidazo[1,2-*a*]pyridine-6-carboxamide (example 8-1)** |
| 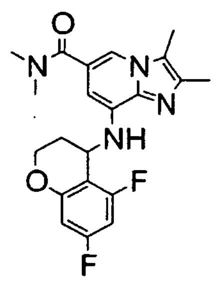 | white solid<br>$^1$H-NMR (270 MHz, $CDCl_3$) δ: 7.45 (d, *J* = 1.3 Hz, 1 H), 6.45-6.35 (m, 2H), 6.26 (s, 1 H), 5.39 (d, *J* = 5.9 Hz, 1 H), 4.88-4.83 (m, 1 H), 4.32-4.28 (m, 2H), 3.13 (s, 6H), 2.36 (s, 6H), 2.29-2.23 (m, 1 H), 2.06-1.93 (m, 1 H) ppm.<br>MS m/z: 401 $(M + H)^+$.<br>**Resolution condition**<br>Column: CHIRALCEL® OD-H (20 mm I.D. x 250 mm, DAICEL)<br>Mobile phase: *n*-Hexane / Ethanol / Diethylamine (90/10/0.1)<br>Flow rate: 18.9 mL/min<br>**(-)-isomer (example 8-2):** optical rotation: $[\alpha]_D^{24}$ = -50.4 ° (c = 1.00, Methanol), retention time: 10 min<br>**(+)-isomer (example 8-3):** optical rotation: $[\alpha]_D^{24}$ = +47.9 ° (c = 1.00, Methanol), retention time: 13 min |

(continued)

| | |
|---|---|
| **Example 9** | **8-[(5,7-Difluoro-3,4-dihydro-2*H*-chromen-4-yl)amino]-*N*-(2-hydroxyethyl)-*N*,2,3-trimethylimidazo[1,2-*a*]pyridine-6-carboxamide (example 9-1)** |
| 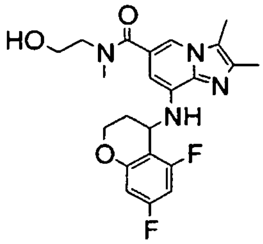 | white solid<br>$^1$H-NMR (270 MHz, $CDCl_3$) δ: 7.53 (s, 1H), 6.51-6.23 (m, 2H), 6.30 (s, 1H), 5.38 (d, *J* = 5.9 Hz, 1 H), 4.95-4.79 (m, 1H), 4.39-4.22 (m, 2H), 4.02-3.83 (m, 2H), 3.80-3.62 (m, 2H), 3.18 (s, 3H), 2.36 (s, 6H), 2.33-2.19 (m, 1 H), 2.15-1.91 (m, 1 H) ppm. (OH was not observed)<br>MS m/z: 431 $(M + H)^+$.<br>**Resolution condition**<br>Column: CHIRALPAK® OD-H (20 mm I.D. x 250 mm, DAICEL)<br>Mobile phase: *n*-Hexane / Ethanol / Diethylamine (90/10/0.1)<br>Flow rate: 18.9 mUmin<br>**(-)-isomer (example 9-2):** optical rotation: $[\alpha]_D^{23}$ = -44.8 ° (c = 0.50, Methanol), retention time: 11 min<br>**(+)-isomer (example 9-3):** optical rotation: $[\alpha]_D^{23}$ = +39.3 ° (c = 0.50, Methanol), retention time: 13 min |
| **Example 10** | **8-[(5-Fluoro-3,4-dihydro-2*H*-chromen-4-yl)amino]-*N*,*N*,2,3-tetramethylimid azo[1,2-*a*]pyridine-6-carboxamide (example 10-1)** |
| 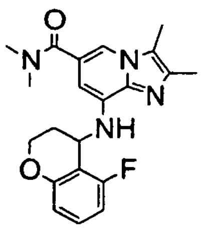 | white solid<br>$^1$H-NMR (270 MHz, $CDCl_3$) δ: 7.45 (s, 1H), 7.25-7.12 (m, 1H), 6.69 (d, *J* = 8.1 Hz, 1 H), 6.67-6.57 (m, 1 H), 6.27 (s, 1 H), 5.40 (d, *J* = 5.4 Hz, 1 H), 4.95-4.85 (m, 1H), 4.40-4.25 (m, 2H), 3.13 (s, 6H), 2.36 (s, 6H), 2.35-1.90 (m, 2H) ppm.<br>MS m/z: 383 $(M + H)^+$.<br>**Resolution condition**<br>Column: CHIRALCEL® OD-H (20 mm I.D. x 250 mm, DAICEL)<br>Mobile phase: *n*-Hexane / Ethanol / Diethylamine (75/25/0.1)<br>Flow rate: 20.0 mL/min<br>**(-)-isomer (example 10-2):** optical rotation: $[\alpha]_D^{24}$ = -51.1 ° (c = 1.00, Methanol), retention time: 5 min<br>**(+)-isomer (example 10-3):** optical rotation: $[\alpha]_D^{2}$ = +51.7 ° (c = 1.00, Methanol), retention time: 8 min |
| **Example 11** | **8-[(5-Fluoro-3,4-dihydro-2*H*-chromen-4-yl)amino]-*N*-(2-hydroxyethyl)-*N*,2, 3-trimethylimidazo[1,2-*a*]pyridine-6-carboxamide (example 11-1)** |
| 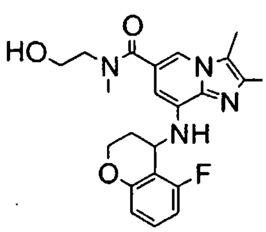 | white solid<br>$^1$H-NMR (300 MHz, $CDCl_3$) δ: 7.52 (s, 1H), 7.18 (dd, *J* = 8.1, 6.6 Hz, 1H), 6.68 (d, *J* = 8.1 Hz, 1H), 6.66-6.56 (m, 1H), 6.31 (s, 1 H), 5.42 (d, *J* = 5.9 Hz, 1H), 4.99-4.85 (m, 1H), 4.38-4.22 (m, 2H), 4.03-3.82 (m, 2H), 3.81-3.63 (m, 2H), 3.18 (s, 3H), 2.36 (s, 6H), 2.32-2.19 (m, 1H), 2.12-1.94 (m, 1 H) ppm. (OH was not observed)<br>MS m/z: 413 $(M + H)^+$.<br>**Resolution condition**<br>Column: CHIRALCEL® AD-H (20 mm I.D. x 250 mm, DAICEL)<br>Mobile phase: *n*-Hexane / Ethanol / Diethylamine (90 /10 /0.1)<br>Flow rate: 18.9 mL/min<br>**(-)-isomer (example 11-2):** optical rotation: $[\alpha]_D^{24}$ = -49.9 ° (c = 0.50, Methanol), retention time: 12 min<br>**(+)-isomer (example 11-3):** optical rotation: $[\alpha]_D^{24}$ = +41.2 ° (c = 0.50, Methanol), retention time: 16 min |

(continued)

| | |
|---|---|
| **Example 12** | **8-[(6-Fluoro-3,4-dihydro-2*H*-chromen-4-yl)amino]-*N*,*N*,2,3-tetramethylimidazo[1,2-*a*]pyridine-6-carboxamide (example 12-1)** |
| 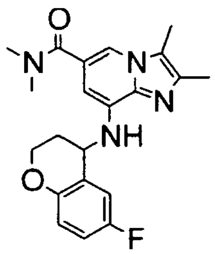 | white solid<br>$^1$H-NMR (270 MHz, $CDCl_3$) δ: 7.48-7.43 (m, 1 H), 7.03 (dd, *J* = 9.2, 3.3 Hz, 1 H), 6.98-6.86 (m, 1H), 6.81 (dd, *J* = 9.2, 4.6 Hz, 1 H), 6.20 (s, 1H), 5.48 (d, *J* = 7.9 Hz, 1H), 4.81-4.68 (m, 1H), 4.33-4.18 (m, 2H), 3.10 (s, 6H), 2.37 (s, 6H), 2.30-2.12 (m, 2H) ppm.<br>MS m/z: 383 $(M + H)^+$.<br>**Resolution condition**<br>Column: CHIRALCEL® OD-H (20 mm I.D. x 250 mm, DAICEL)<br>Mobile phase: *n*-Hexane / Ethanol / Diethylamine (80 / 20 / 0.1)<br>Flow rate: 18.9 mL/min<br>**(-)-isomer (example 12-2):** optical rotation: $[\alpha]_D^{24}$ = -3.4 ° (c = 1.00, Methanol), retention time: 9 min<br>**(+)-isomer (example 12-3):** optical rotation: $[\alpha]_D^{24}$ = +2.4 ° (c = 1.00, Methanol), retention time: 6 min |
| **Example 13** | **8-[(6-Fluoro-3,4-dihydro-2*H*-chromen-4-yl)amino]-*N*-(2-hydroxyethyl)-*N*,2, 3-trimethylimidazo[1,2-a]pyridine-6-carboxamide (example 13-1)** |
| 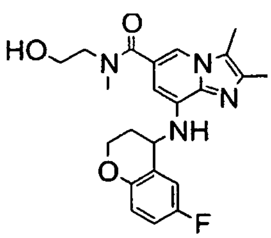 | white solid<br>$^1$H-NMR (300MHz, $CDCl_3$) δ: 7.53 (s, 1H), 7.03 (dd, *J* = 8.1, 2.9 Hz, 1 H), 6.95-6.88 (m, 1H), 6.83-6.78 (m, 1H), 6.24 (s, 1 H), 5.52 (d, *J* = 6.5 Hz, 1H), 4.90-4.70 (m, 1H), 4.32-4.21 (m, 2H), 3.98-3.83 (m, 2H), 3.79-3.65 (m, 2H), 3.14 (s, 3H), 2.37 (s, 6H), 2.27-2.13 (m, 2H) ppm. (OH was not observed)<br>MS m/z: 413 $(M + H)^+$.<br>**Resolution condition**<br>Column: CHIRALCEL® OD-H (20 mm I.D. x 250 mm, DAICEL)<br>Mobile phase: n-Hexane / Ethanol / Diethylamine 92 / 10 / 0.1<br>Flow rate: 18.9 mL/min<br>**(-)-isomer (example 13-2):** optical rotation: $[\alpha]_D^{24}$ = -7.0 ° (c = 1.00, Methanol), retention time: 17 min<br>**(+)-isomer (example 13-3):** optical rotation: $[\alpha]_D^{24}$ = +5.2 ° (c = 1.00, Methanol), retention time: 13 min |
| **Example 14** | **8-[(8-Fluoro-3,4-dihydro-2*H*-chromen-4-yl)amino]-*N*,*N*,2,3-tetramethylimidazo[1,2-*a*]pyridine-6-carboxamide (example 14-1)** |
| 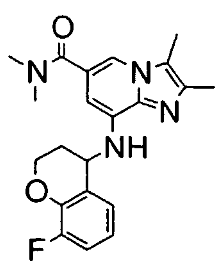 | white solid<br>$^1$H-NMR (300MHz, $CDCl_3$) δ: 7.45 (s, 1H), 7.10-6.99 (m, 2H), 6.84-6.77 (m, 1H), 6.23 (s, 1H), 5.60 (d, *J* = 7.3 Hz, 1H), 4.82-4.77 (m, 1H), 4.44-4.31 (m, 2H), 3.11 (s, 6H), 2.37 (s, 6H), 2.34-2.13 (m, 2H) ppm.<br>MS m/z: 383 $(M + H)^+$.<br>**Resolution condition**<br>Column: CHIRALPAK® AD-H (20 mm I.D. x 250 mm, DAICEL)<br>Mobile phase: *n*-Hexane / Ethanol / Diethylamine (80/20/0.1)<br>Flow rate: 18.9 mL/min<br>**(-)-isomer (example 14-2):** optical rotation: $[\alpha]_D^{24}$ = -13.8 ° (c = 0.30, Methanol), retention time: 9 min<br>**(+)-isomer (example 14-3):** optical rotation: $[\alpha]_D^{24}$ = +9.3 ° (c = 0.30, Methanol), retention time: 13 min |

(continued)

| | |
|---|---|
| **Example 15** | **8-[(8-Fluoro-3,4-dihydro-2*H*-chromen-4-yl)amino]-*N*-(2-hydroxyethyl)-*N*,2, 3-trimethylimidazo[1,2-*a*]pyridine-6-carboxamide (example 15-1)** |
| 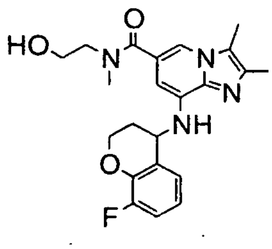 | white solid<br>$^1$H-NMR (270 MHz, $CDCl_3$) δ: 7.54 (s, 1H), 7.10-6.98 (m, 2H), 6.84-6.77 (m, 1H), 6.27 (s, 1H), 5.56 (d, *J* = 7.3 Hz, 1 H), 4.82-4.76 (m, 1H), 4.43-4.31 (m, 2H), 4.01-3.84 (m, 2H), 3.76-3.65 (m, 2H), 3.15 (s, 3H), 2.36 (s, 6H), 2.32-2.20 (m, 2H) ppm. (OH was not observed)<br>MS m/z 413: $(M + H)^+$.<br>**Resolution condition**<br>Column: CHIRALPAK® AD-H (20 mm I.D. x 250 mm, DAICEL)<br>Mobile phase: *n*-Hexane / Ethanol / Diethylamine (80 / 20 / 0.1)<br>Flow rate: 18.9 mL/min<br>(-)-isomer (example 15-2): optical rotation: $[\alpha]_D^{24}$ = -8.4 ° (c = 1.00, Methanol), retention time: 8 min<br>**(+)-isomer (example 15-3):** optical rotation: $[\alpha]_D^{24}$ = +6.7 ° (c = 1.00, Methanol), retention time: 11 min |
| **Example 16** | ***N*-(2-Hydroxyethyl)-*N*,2,3-trimethyl-8-[(7-methyl-3,4-dihydro-2*H*-chromen-4-yl)amino]imidazo[1,2-*a*]pyridine-6-carboxamide (example 16-1)** |
| 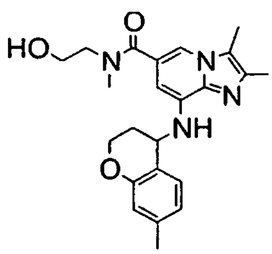 | white solid<br>$^1$H-NMR (300 MHz, $CDCl_3$) δ: 7.52 (s, 1H), 7.17 (d, *J* = 7.3 Hz, 1H), 6.77-6.61 (m, 2H), 6.24 (s, 1 H), 5.48 (d, *J* = 7.3 Hz, 1H), 4.78-4.66 (m, 1H), 4.29-4.20 (m, 2H), 4.00-3.82 (m, 2H), 3.82-3.61 (m, 2H), 3.15 (s, 3H), 2.36 (s, 6H), 2.30 (s, 3H), 2.42-2.09 (m, 2H) ppm. (OH was not observed)<br>MS m/z: 409 $(M + H)^+$.<br>**Resolution condition**<br>Column: CHIRALPAK® AD-H (20 mm I.D. x 250 mm, DAICEL)<br>Mobile phase: n-Hexane / Ethanol / Diethylamine (87/13/0.1)<br>Flow rate: 18.9 mL/min<br>**(-)-isomer (example 16-2):** optical rotation: $[\alpha]_D^{24}$ = -12.5 ° (c = 1.00, Methanol), retention time: 13 min<br>**(+)-isomer (example 16-3):** optical rotation: $[\alpha]_D^{24}$ = +11.7 ° (c = 1.00, Methanol), retention time: 15 min |
| **Example 17** | ***N*,*N*,2,3-Tetramethyl-8-[(5-methyl-3,4-dihydro-2H-chromen-4-yl)amino]imi dazo[1,2-*a*]pyridine-6-carboxamide (example 17-1)** |
| 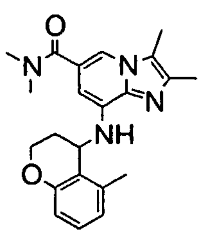 | white solid<br>$^1$H-NMR (300 MHz, $CDCl_3$) δ: 7.45 (s, 1H), 7.17-7.07 (m, 1H), 6.80-6.69 (m, 2H), 6.26 (s, 1H), 5.44-5.27 (m, 1H), 4.73-4.59 (m, 1H), 4.36-4.15 (m, 2H), 3.14 (s, 6H), 2.36 (s, 3H), 2.35 (s, 3H), 2.21 (s, 3H), 2.31-1.95 (m, 2H) ppm.<br>MS m/z: 379 $(M + H)^+$.<br>**Resolution condition**<br>Column: CHIRALCEL® AD-H (20 mm I.D. x 250 mm, DAICEL)<br>Mobile phase: *n*-Hexane / Ethanol / Diethylamine (75/25/0.1)<br>Flow rate: 18.9 mL/min<br>**(-)-isomer (example 17-2):** optical rotation: $[\alpha]_D^{24}$ -4.6 ° (c = 1.00, Methanol), retention time: 5 min<br>**(+)-isomer (example 17-3):** optical rotation: $[\alpha]_D^{24}$ = +4.7 ° (c = 1.00, Methanol), retention time: 9 min |

(continued)

| Example 18 | **8-[(5-Chloro-3,4-dihydro-2*H*-chromen-4-yl)amino]-*N*,*N*,2,3-tetramethylimi dazo[1,2-a]pyridine-6-carboxamide (example 18-1)** |
|---|---|
| 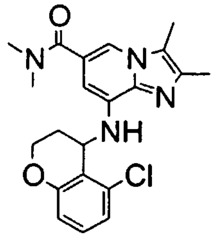 | white solid<br>$^1$H-NMR (300 MHz, $CDCl_3$) δ: 7.46 (s, 1H), 7.36-7.17 (m, 2H), 6.89-6.76 (m, 1H), 6.22 (s, 1H), 5.54-5.40 (m, 1H), 4.87-4.70 (m, 1H), 4.52-4.32 (m, 2H), 3.11 (s, 6H), 2.37 (s, 6H), 2.51-2.05 (m, 2H) ppm.<br>MS m/z: 399 $(M + H)^+$.<br>**Resolution condition**<br>Column: CHIRALPAK® AD-H (20 mm I.D. x 250 mm, DAICEL)<br>Mobile phase: n-Hexane / Ethanol / Diethylamine (85 / 15 / 0.1)<br>Flow rate: 18.9 mL/min<br>**(-)-isomer (example 18-2):** optical rotation: $[\alpha]_D^{24}$ = -40.8 ° (c = 1.00, Methanol), retention time: 15 min<br>**(+)-isomer (example 18-3):** optical rotation: $[\alpha]_D^{24}$ = +43.1 ° (c = 1.00, Methanol), retention time: 20 min |
| Example 19 | **8-[(5-Chloro-3,4-dihydro-2*H*-chromen-4-yl)amino]-*N*-(2-hydroxyethyl)-*N*,2, 3-trimethylimidazo[1,2-*a*]pyridine-6-carboxamide (example 19-1)** |
| 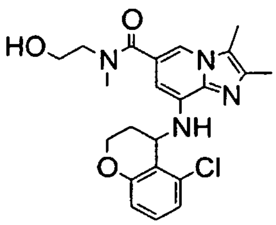 | white solid<br>$^1$H-NMR (300 MHz, $CDCl_3$) δ: 7.54 (s, 1H), 7.38-7.18 (m, 2H), 6.90-6.75 (m, 1H), 6.26 (s, 1H), 5.47 (d, *J* = 6.6 Hz, 1 H), 4.88-4.71 (m, 1H), 4.50-4.30 (m, 2H), 4.04-3.81 (m, 2H), 3.81-3.60 (m, 2H), 3.14 (s, 3H), 2.36 (s, 6H), 2.46-2.12 (m, 2H) ppm. (OH was not observed)<br>MS m/z: 429 $(M + H)^+$.<br>**Resolution condition**<br>Column: CHIRALPAK® AD-H (20 mm I.D. x 250 mm, DAICEL)<br>Mobile phase: *n*-Hexane / Ethanol / Diethylamine (85/15/0.1)<br>Flow rate: 18.9 mL/min<br>**(-)-isomer (example 19-2):** optical rotation: $[\alpha]_D^{24}$ = -36.4 ° (c = 1.00, Methanol), retention time: 14 min<br>**(+)-isomer (example 19-3):** optical rotation: $[\alpha]_D^{24}$= +36.6 ° (c = 1.00, Methanol), retention time: 17 min |

All publications, including but not limited to, issued patents, patent applications, and journal articles, cited in this application are each herein incorporated by reference in their entirety.

Although the invention has been described above with reference to the disclosed embodiments, those skilled in the art will readily appreciate that the specific experiments detailed are only illustrative of the invention. It should be understood that various modifications could be made without departing from the spirit of the invention.

## Claims

**1.** A compound of the formula (1):

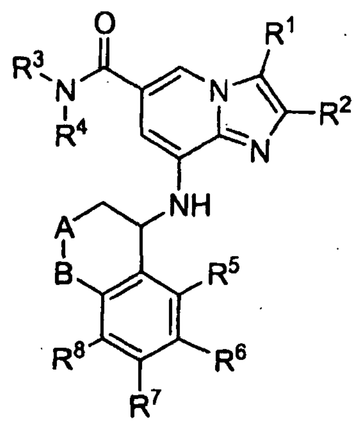

(I)

or a pharmaceutically acceptable salt thereof, wherein:

-A-B- represents $-O-CH_2-$, $-S-CH_2-$, $-CH_2O-$ or $-CH_2-S-$;
$R^1$ represents a $C_1$-$C_6$ alkyl group being unsubstituted or substituted with 1 to 2 substituents independently selected from the group consisting of a hydroxy group, a moiety convertible in vivo into a hydroxy group and a $C_1$-$C_6$ alkoxy group;
$R^2$ represents a $C_1$-$C_6$ alkyl group;
$R^3$ represents a $C_1$-$C_6$ alkyl group, $C_3$-$C_7$ cycloalkyl group or $C_3$-$C_7$cycloalkyl $C_1$-$C_6$ alkyl group;
$R^4$ represents a $C_1$-$C_8$ alkyl group being unsubstituted or substituted with 1 to 3 substituents independently selected from the group consisting of a halogen atom, a hydroxy group, a moiety convertible in vivo into a hydroxy group and a $C_1$-$C_6$ alkoxy group; and
$R^5$, $R^6$, $R^7$and $R^8$ independently represent a hydrogen atom, a halogen atom or a $C_1$-$C_6$ alkyl group wherein the moiety convertible in vivo into a hydroxy group is a $C_1$-$C_6$ alkyl carbonyl oxy group or a $C_1$-$C_6$ alkyl carbonyl oxy methyl oxy group.

**2.** The compound or the pharmaceutically acceptable salt, as claimed in claim 1, wherein
A-B- is $-O-CH_2-$or $-CH_2-O-$;
$R^1$, $R^2$ and $R^3$ are independently a $C_1$-$C_6$ alkyl group;
$R^4$ is a $C_1$-$C_6$ alkyl group being unsubstituted or substituted with 1 substituent selected from the group consisting of a hydroxy group and a $C_1$-$C_6$ alkoxy group;
$R^5$ is a hydrogen atom, a fluorine atom or a $C_1$-$C_6$ alkyl group;
$R^7$ is a hydrogen atom, a halogen atom or a $C_1$-$C_6$ alkyl group; and
$R^6$ and $R^8$ are independently a hydrogen atom, a halogen atom or a $C_1$-$C_6$ alkyl group.

**3.** The compound or the pharmaceutically acceptable salt, as claimed in claim 1, wherein
-A-B- is $-CH_2-O-$;
$R^1$ $R^2$ and $R^3$ are each methyl group;
$R^4$ is a $C_1$-$C_2$ alkyl group being unsubstituted or substituted with a hydroxy group;
$R^5$ and $R^7$ are independently a hydrogen atom or methyl group; and
$R^6$ and $R^8$are each hydrogen atom.

**4.** The compound of claim 1, which is selected from:
(+)-*N*,*N*,2,3-Tetramethyl-8-[(5-methyl-3,4-dihydro-2*H*-chromen-4-yl)amino]imidazo[1,2-*a*]pyridine-6-carbox amide; or a pharmaceutically acceptable salt thereof.

**5.** A pharmaceutical composition comprising the compound or the pharmaceutically acceptable salt thereof as claimed in any one of claims 1 to 4, and a Pharmaceutical acceptable carrier.

**6.** The pharmaceutical composition as claimed in claim 5 further comprising other pharmacologically active agent(s).

**7.** A use of the compound of formula (I) or the pharmaceutically acceptable salt thereof, as claimed in any one of claims 1 to 4, for the manufacture of a medicament for the treatment of a condition mediated by acid pump inhibitory activity.

**8.** The use as claimed in claim 7, wherein said condition is gastrointestinal disease, gastroesophageal disease, gastroesophageal reflux disease (GERD), peptic ulcer, gastric ulcer, duodenal ulcer, NSAID-induced ulcers, gastritis, infection of Helicobacter pylori, dyspepsia, functional dyspepsia, Zollinger-Ellison syndrome, non-erosive reflux disease (NERD), visceral pain, heartburn, nausea, esophagitis, dysphagia, hypersalivation, airway disorders or asthma.

**Patentansprüche**

**1.** Verbindung der Formel (I):

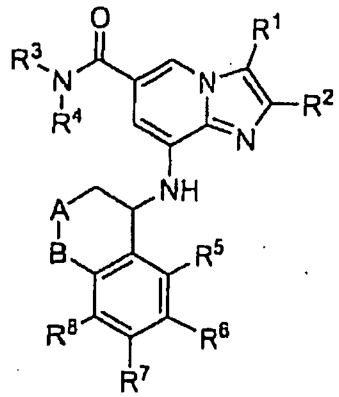

(I)

oder ein pharmazeutisch verträgliches Salz davon, worin:

-A-B- für -O-$CH_2$-, -S-$CH_2$-, -$CH_2$-O- oder -$CH_2$-S- steht; $R^1$ für eine $C_1$-$C_6$-Alkylgruppe, die unsubstituiert ist oder mit 1 bis 2 Substituenten, unabhängig ausgewählt aus der Gruppe, bestehend aus einer Hydroxygruppe, einer Gruppierung, die in vivo in eine Hydroxygruppe umwandelbar ist, und einer $C_1$-$C_6$-Alkoxygruppe, substituiert ist, steht;
$R^2$ für eine $C_1$-$C_6$-Alkylgruppe steht;
$R^3$ für eine $C_1$-$C_6$-Alkylgruppe, $C_3$-$C_7$-Cycloalkylgruppe oder $C_3$-$C_7$-Cycloalkyl-$C_1$-$C_6$-alkylgruppe steht;
$R^4$ für eine $C_1$-$C_6$-Alkylgruppe steht, die unsubstituiert ist oder mit 1 bis 3 Substituenten, unabhängig ausgewählt aus der Gruppe, bestehend aus einem Halogenatom, einer Hydroxygruppe, einer Gruppierung, die in vivo in eine Hydroxygruppe umwandelbar ist, und einer $C_1$-$C_6$-Alkoxygruppe, substituiert ist; und
$R^5$, $R^6$, $R^7$ und $R^8$ unabhängig für ein Wasserstoffatom, ein Halogenatom oder eine $C_1$-$C_6$-Alkylgruppe stehen, wobei die in vivo in eine Hydroxygruppe umwandelbare Gruppierung eine $C_1$-$C_6$-Alkylcarbonyloxygruppe oder eine $C_1$-$C_6$-Alkylcarbonyloxy-methyloxygruppe ist.

**2.** Verbindung oder das pharmazeutisch verträgliche Salz nach Anspruch 1, wobei
A-B- für -O-$CH_2$- oder -$CH_2$-O- steht;
$R^1$, $R^2$ und $R^3$ unabhängig eine $C_1$-$C_6$-Alkylgruppe sind;
$R^4$ eine $C_1$-$C_6$-Alkylgruppe ist, die unsubstituiert ist oder mit einem Substituenten, ausgewählt aus der Gruppe, bestehend aus einer Hydroxygruppe und einer $C_1$-$C_6$-Alkoxygruppe, substituiert ist;
$R^5$ ein Wasserstoffatom, ein Fluoratom oder eine $C_1$-$C_6$-Alkylgruppe ist;
$R^7$ ein Wasserstoffatom, ein Halogenatom oder eine $C_1$-$C_6$-Alkylgruppe ist und
$R^6$ und $R^8$ unabhängig ein Wasserstoffatom, ein Halogenatom oder eine $C_1$-$C_6$-Alkylgruppe sind.

**3.** Verbindung oder das pharmazeutisch verträgliche Salz nach Anspruch 1, wobei
-A-B- -$CH_2$-O- ist;
$R^1$, $R^2$ und $R^3$ jeweils eine Methylgruppe sind;
$R^4$ eine $C_1$-$C_2$-Alkylgruppe ist, die unsubstituiert ist oder mit einer Hydroxygruppe substituiert ist;
$R^5$ und $R^7$ unabhängig ein Wasserstoffatom oder eine Methylgruppe sind und
$R^6$ und $R^8$ jeweils ein Wasserstoffatom sind.

**4.** Verbindung nach Anspruch 1, ausgewählt aus:

(+)-*N, N*, 2,3- Tetramethyl- 8-[(5- methyl- 3,9- dihydro- 2*H*-chromen- 4- yl) amino] imidazo [1,2-*a*] pyridin- 6- carboxamid

und einem pharmazeutisch verträglichen Salz davon.

**5.** Pharmazeutische Zusammensetzung, umfassend die Verbindung oder das pharmazeutisch verträgliche Salz davon nach einem der Ansprüche 1 bis 4 und einen pharmazeutisch verträglichen Träger.

**6.** Pharmazeutische Zusammensetzung nach Anspruch 5, die außerdem ein anderes pharmakologisch aktives Mittel (andere pharmakologisch aktive Mittel) umfasst.

**7.** Verwendung der Verbindung der Formel (I) oder des pharmazeutisch verträglichen Salzes davon nach einem der Ansprüche 1 bis 4 zur Herstellung eines Medikaments für die Behandlung eines Zustandes, der durch Säurepumpeninhibitoraktivität vermittelt wird.

**8.** Verwendung nach Anspruch 7, wobei der Zustand gastrointestinale Erkrankung, gastroösophageale Erkrankung, gastroösophageale Refluxerkrankung (GERD), peptischer Ulcus, Magenulcus, Duodenalulcus, NSAID-induzierte Ulcera, Gastritis, Infektion durch Helicobacter pylori, Dyspepsie, funktionelle Dyspepsie, Zollinger-Ellison-Syndrom, nicht-erosive Refluxerkrankung (NERD), visceraler Schmerz, Sodbrennen, Nausea, Ösophagitis, Dysphagie, Hypersalivation, Atemwegserkrankungen oder Asthma ist..

**Revendications**

**1.** Composé de formule (I) :

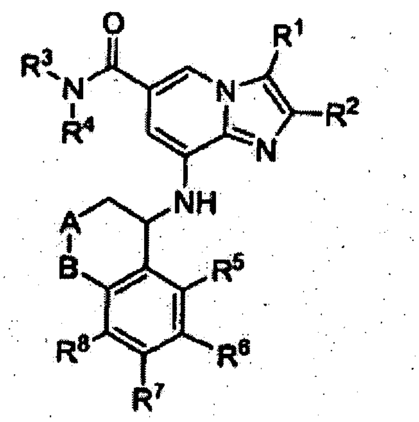

(I)

ou un de ses sels pharmaceutiquement acceptables, formule dans laquelle :

-A-B- représente un groupe -O-$CH_2$-, S-$CH_2$-, -$CH_2$-O- ou -$CH_2$-S- ;
$R^1$ représente un groupe alkyle en $C_1$ à $C_6$ qui est non substitué ou substitué avec 1 ou 2 substituants choisis indépendamment dans le groupe consistant en un groupe hydroxy, un groupement pouvant être converti in vivo en un groupe hydroxy et un groupe alkoxy en $C_1$ à $C_6$ ;
$R^2$ représente un groupe alkyle en $C_1$ à $C_6$ ;
$R^3$ représente un groupe alkyle en $C_1$ à $C_6$, un groupe cycloalkyle en $C_3$ à $C_7$ ou un groupe (cycloalkyle en $C_3$ à $C_7$) (alkyle en $C_1$ à $C_6$) ;
$R^4$ représente un groupe alkyle en $C_1$ à $C_6$ qui est non substitué ou substitué avec 1 à 3 substituants choisis indépendamment dans le groupe consistant en un atome d'halogène, un groupe hydroxy, un groupement pouvant être converti in vivo en un groupe hydroxy et un groupe alkoxy en $C_1$ à $C_6$ ; et
$R^5$, $R^6$, $R^7$ et $R^8$ représentent indépendamment un atome d'hydrogène, un atome d'halogène ou un groupe alkyle en $C_1$ à $C_6$, où le groupement pouvant être converti in vivo en un groupe hydroxy est un groupe (alkyle en $C_1$ à $C_6$)carbonyloxy ou un groupe (alkyle en $C_1$ à $C_6$)carbonyloxyméthyloxy.

**2.** Composé ou sel pharmaceutiquement acceptable suivant la revendication 1, dans lequel

-A-B- représente un groupe $-O-CH_2-$ ou $-CH_2-O-$ ;
$R^1$, $R^2$ et $R^3$ représentent indépendamment un groupe alkyle en $C_1$ à $C_6$ ;
$R^4$ représente un groupe alkyle en $C_1$ à $C_6$ qui est non substitué ou substitué avec 1 substituant choisi dans le groupe consistant en un groupe hydroxy et un groupe alkoxy en $C_1$ à $C_6$ ;
$R^5$ représente un atome d'hydrogène, un atome de fluor ou un groupe alkyle en $C_1$ à $C_6$ ;
$R^7$ représente un atome d'hydrogène, un atome d'halogène ou un groupe alkyle en $C_1$ à $C_6$ ; et
$R^6$ et $R^8$ représentent indépendamment un atome d'hydrogène, un atome d'halogène ou un groupe alkyle en $C_1$ à $C_6$.

**3.** Composé ou sel pharmaceutiquement acceptable suivant la revendication 1, dans lequel

-A-B- représente un groupe $-CH_2-O-$ ;
$R^1$, $R^2$ et $R^3$ représentent chacun un groupe méthyle ;
$R^4$ représente un groupe alkyle en $C_1$ ou $C_2$ qui est non substitué ou substitué avec un groupe hydroxy ;
$R^5$ et $R^7$ représentent indépendamment un atome d'hydrogène ou un groupe méthyle ; et
$R^6$ et $R^8$ représentent chacun un atome d'hydrogène.

**4.** Composé suivant la revendication 1, qui est choisi enture :

le (+)-*N*,*N*,2,3-tétraméthyl-8-[(5-méthyl-3,4-dihydro-2*H*-chromèn-4-yl)amino]imidazo[1,2-a]pyridine-6-carboxamide ;

et un de ses sels pharmaceutiquement acceptables.

**5.** Composition pharmaceutique comprenant le composé ou son sel pharmaceutiquement acceptable suivant l'une quelconque des revendications 1 à 4 et un support pharmaceutiquement acceptable.

**6.** Composition pharmaceutique suivant la revendication 5, comprenant en outre un ou plusieurs autres agents pharmacologiquement actifs.

**7.** Utilisation du composé de formule (I) ou de son sel pharmaceutiquement acceptable suivant l'une quelconque des revendications 1 à 4, pour la production d'un médicament destiné au traitement d'une affection à médiation par l'activité inhibitrice de la pompe d'acide.

**8.** Utilisation suivant la revendication 7, dans laquelle ladite affection est une maladie gastro-intestinale, une maladie gastro-oesophagienne, la maladie de reflux gastro-oesophagien (GERD), l'ulcère peptique, l'ulcère gastrique, l'ulcère duodénal, les ulcères induits par les médicaments anti-inflammatoires non stéroïdiens, la gastrite, une infection par Helicobacter pylori, la dyspepsie, la dyspepsie fonctionnelle, le syndrome de Zollinger-Ellison, la maladie de reflux non érosive (NERD), la douleur viscérale, les brûlures d'estomac, les nausées, l'oesophagite, la dysphagie, l'hypersalivation, des troubles des voies aériennes ou l'asthme.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9955706 A
- WO 04046144 A
- WO 0220523 A
- WO 00078751 A
- WO 02020523 A
- US 6106864 A
- WO 0035298 A
- WO 9111172 A
- WO 9402518 A
- WO 9855148 A
- WO 0078751 A
- WO 04024081 A


### Non-patent literature cited in the description


- **SACHS, G.** *Digestive Diseases and Sciences,* 1995, vol. 40, 3S-23S
- **SACHS.** *Annu Rev Pharmacol Toxicol,* 1995, vol. 35, 277-305
- **POPE, A. ; PARSONS, M.** *Trends in Pharmacological Sciences,* 1993, vol. 14, 323-5
- **VAKIL, N.** *Alimentary Pharmacology and Therapeutics,* 2004, vol. 19, 1041-1049
- **KILJANDER, TONI O.** *American Journal of Medicine,* 2003, vol. 115 (3A), 65S-71S
- **STAHL ; WERMUTH.** Handbook of Pharmaceutical Salts: Properties, Selection, and Use. Wiley-VCH, 2002
- **HALEBLIAN.** *J Pharm Sci,* August 1975, vol. 64 (8), 1269-1288
- Protective Groups in Organic Synthesis. John Wiley & Sons, 1999
- **T. W. GREENE et al.** *Protective Groups in Organic Synthesis,* 1999, 369-453
- Remington's Pharmaceutical Sciences. Mack Publishing Company, 1995
- **LIANG ; CHEN.** *Expert Opinion in Therapeutic Patents,* 2001, vol. 11 (6), 981-986
- **H. LIEBERMAN ; L. LACHMAN.** Pharmaceutical Dosage Forms: Tablets. Marcel Dekker, 1980, vol. 1
- **VERMA et al.** *Pharmaceutical Technology On-line,* 2001, vol. 25 (2), 1-14
- **FINNIN ; MORGAN.** *J Pharm Sci,* October 1999, vol. 88 (10), 955-958
- *Biochemical Pharmacology,* 1988, vol. 37, 2231-2236
- **WATANABE K et al.** *J. Physiol. (Paris,* 2000, vol. 94, 111-116
- **HEIDENHAIN R.** *Arch Ges Physiol.,* vol. 19, 148-167
- **SHIYIN YEE.** *Pharmaceutical Research,* 1997, 763
- **Z.ZHOU et al.** *Biophysical journal,* 1998, vol. 74, 230-241
- *Tetrahedron Asym.,* 1997, vol. 8, 3059